# EUROPÄISCHE PATENTSCHRIFT

(11) **EP 0 944 608 B1**
(45) Veröffentlichungstag und Bekanntmachung des Hinweises auf die Patenterteilung: **16.04.2003**
(21) Anmeldenummer: 97951213.4
(22) Anmeldetag: 17.11.1997
(51) Int. Cl.: C07D 263/14, A01N 43/76, C07D 263/10, C07D 413/10, C07C 233/66

(54) **4-CYCLOHEXYLPHENYL-OXAZOLINE UND IHRE VERWENDUNG ZUR BEKÄMPFUNG VON TIERISCHEN SCHÄDLINGEN**
4-CYCLOHEXYLPHENYL-OXAZOLINES AND THEIR USE FOR CONTROLLING ANIMAL PESTS
4-CYCLOHEXYLPHENYLOXAZOLINES ET LEUR UTILISATION POUR COMBATTRE LES PARASITES ANIMAUX

(30) Priorität: 28.11.1996 DE 19649307
(43) Veröffentlichungstag der Anmeldung: 29.09.1999
(73) Patentinhaber: Bayer CropScience AG, 40789 Monheim (DE)
(72) Erfinder: KRÄMER, Wolfgang, D-51399 Burscheid (DE); ERDELEN, Christoph, D-42799 Leichlingen (DE); WACHENDORFF-NEUMANN, Ulrike, D-56566 Neuwied (DE); TURBERG, Andreas, D-40699 Erkrath (DE); MENCKE, Norbert, D-51381 Leverkusen (DE)
(86) Internationale Anmeldenummer: EP9706392
(87) Internationale Veröffentlichungsnummer: WO98023600

(56) Entgegenhaltungen:
- EP-A- 0 432 661
- EP-A- 0 645 085
- EP-A- 0 696 584

## Beschreibung

Die vorliegende Erfindung betrifft neue 4-Cyclohexylphenyl-oxazoline, Verfahren zu ihrer Herstellung und ihre Verwendung zur Bekämpfung von tierischen Schädlingen.

Es ist bereits bekannt, daß bestimmte substituierte Biphenyl-oxazoline insektizide und akarizide Eigenschaften aufweisen (vgl. z.B. EP-A-0 432 661 oder EP-A-0 696 584).

In EP-A-0 645 085 werden phenylsubstituiere Oxa- und Thiazole sowie die Verwendung als Akarizide beschrieben.

In EP-A-0 432 661 werden 2-substituierte Phenyl-2-oxazoline und ihre Verwendung als Insektizide und Akarizide beschrieben.

Die Wirkungshöhe und/oder Wirkungsdauer dieser bekannten Verbindungen ist jedoch, insbesondere gegen bestimmte Organismen oder bei niedrigen Anwendungskonzentrationen nicht in allen Anwendungsgebieten völlig zufriedenstellend.

Es wurden neue 4-Cyclohexylphenyl-oxazoline der Formel (I) gefunden, 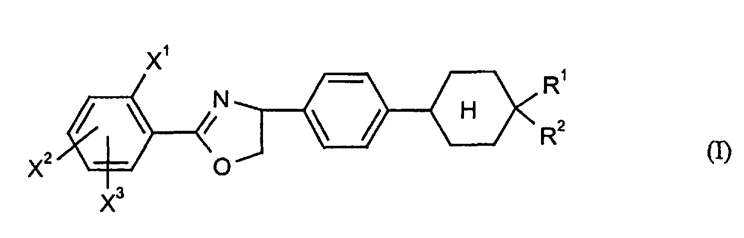 in welcher
- X¹: für Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy, Amino, C₁-C₄-Alkylamino oder Di(C₁-C₄-alkyl)amino (insbesondere für Fluor oder Chlor, hervorgehoben für Fluor) steht,
- X² und X³: gleich oder verschieden sind und für Wasserstoff, Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy, Amino, C₁-C₄-Alkylamino oder Di(C₁-C₄-alkyl)amino stehen,
- R¹: für Wasserstoff, -OR³ oder -SR³ steht und
- R²: für den Rest -OR⁴ steht
oder
- R¹ und R²: gemeinsam für Sauerstoff, für =N-OR⁵ oder =N-NR⁶R⁷ stehen,
- R³ und R⁴: gleich oder verschieden sind und für Wasserstoff, C₁-C₄-Alkyl oder gegebenenfalls, vorzugsweise im Phenylteil, einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl
oder gemeinsam mit den Heteroatomen, an die sie gebunden sind und dem diese verbindenden C-Atom, für einen gegebenenfalls einfach oder zweifach, gleich oder verschieden substituierten 5- oder 6-gliedrigen Ring (der bevorzugt keine weiteren Heteroatome enthält), wobei als Substituenten gegebenenfalls durch Halogen, C₁-C₆-Alkoxy, C₂-C₆-Alkenyloxy, Benzyloxy oder Phenoxy (welche jeweils im Ring gegebenenfalls einfach bis dreifach, bevorzugt einfach oder zweifach, gleich oder verschieden durch Halogen, C₁-C₈-Alkyl, C₁-C₄-Alkoxy oder Phenoxy substituiert sind) substituiertes
C₁-C₁₈-Alkyl, C₂-C₆-Alkenyl, gegebenenfalls einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino, C₁-C₄-Alkoxy-carbonyl, Phenyl (welches gegebenenfalls einfach oder zweifach, gleich oder verschieden durch Halogen oder C₁-C₄-Alkyl substituiert ist) oder Benzyloxy (welches gegebenenfalls einfach oder zweifach, gleich oder verschieden im Ring durch Halogen oder C₁-C₄-Alkyl substituiert ist) substituiertes Phenyl oder -NR¹²R¹³ in Frage kommen.
R¹² für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil, einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
R¹³ für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Phenyl, Phenylcarbonyl oder Benzyl, für -CONR¹⁴R¹⁵ oder -SO₂NR¹⁴R¹⁵ steht,
R¹⁴ für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
R¹⁵ für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Phenyl, Phenylcarbonyl oder Benzyl steht,
- R⁵: für Wasserstoff, C₁-C₆-Alkyl, C₂-C₆-Alkenyl, C₂-C₆-Alkinyl, C₁-C₄-Halogenalkyl oder gegebenenfalls, vorzugsweise im Phenylteil, einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
- R⁶: für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
- R⁷: für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Phenyl, Phenylcarbonyl oder Benzyl oder für -CONR⁸R⁹ oder -SO₂NR⁸R⁹ steht,
R⁸ für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
R⁹ für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Phenyl, Phenylcarbonyl oder Benzyl steht.

Die 4-Cyclohexylphenyl-oxazoline der Formel (I) können, auch in Abhängigkeit von den Substituenten, als optische und/oder geometrische Isomeren anfallen. Die vorliegende Erfindung betrifft sowohl die Isomerengemische als auch die reinen Isomeren.

Weiterhin wurde gefunden, daß man die 4-Cyclohexylphenyl-oxazoline der Formel (I) erhält, wenn man
a) Chlorethan-Derivate der Formel (II) 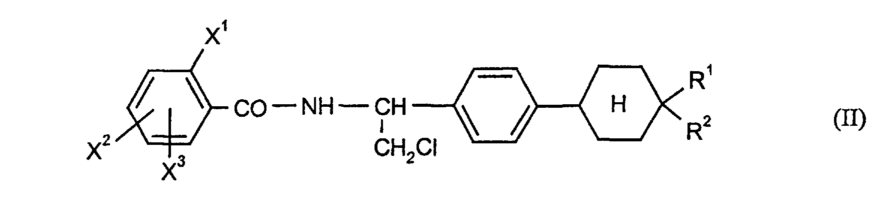 in welcher
   X¹, X², X³, R¹ und R² die oben angegebene Bedeutung haben,
   in Gegenwart einer Base, gegebenenfalls in Gegenwart eines Katalysators und gegebenenfalls in Gegenwart eines Verdünnungsmittels cyclisiert;
   oder
b) nach dem Verfahren (a) erhältliche 4-Cyclohexylphenyl-oxazoline der Formel (Ia) 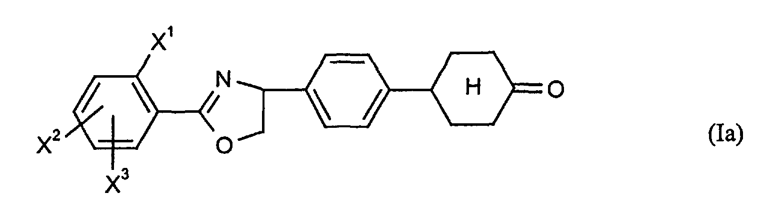 in welcher
   X¹, X² und X³ die oben angegebene Bedeutung haben,
   in üblicher und allgemein bekannter Art und Weise derivatisiert zu
(b1) Ketalen
   durch Umsetzung mit Alkoholen der Formel (III), Thioalkoholen der Formel (IV) oder Verbindungen der Formel (V)

   HOR¹⁰ (III)

   HSR¹⁰ (IV)

   HO-A-O(S)H (V)

   in welchen
   - R¹⁰: für die oben genannten Bedeutungen von R³ und/oder R⁴ steht und
   - A: für eine gegebenenfalls substituierte C₂- oder C3-Kohlenwasserstoffkette steht,
   gegebenenfalls in Gegenwart eines Katalysators und gegebenenfalls in Gegenwart eines Verdünnungsmittels;
   oder
(b2) Oximen und Oximethem
   durch Umsetzung mit Hydroxylaminen der Formel (VI)

   H₂N-O-R⁵ (VI)

   in welcher
   - R⁵: die oben angegebene Bedeutung hat
   oder mit deren Salzen, wie beispielsweise Halogenwasserstoff-Addukten gegebenenfalls in Gegenwart eines Verdünnungsmittels und gegebenenfalls in Gegenwart einer Base umsetzt; wobei es auch möglich ist, die entstehenden Derivate mit R⁵ = Wasserstoff nachträglich zu alkylieren;
   oder
(b3) Hydrazonen
   durch Umsetzung mit Hydrazinen der Formel (VII)

   H₂N-NR⁶R⁷ (VII)

   in welcher
   - R⁶ und R⁷: die oben angegebene Bedeutung haben,
   gegebenenfalls in Gegenwart eines Verdünnungsmittels;
   oder
c) nach dem Verfahren (a) erhältliche 4-Cyclohexylphenyl-oxazoline der Formel (Ib) 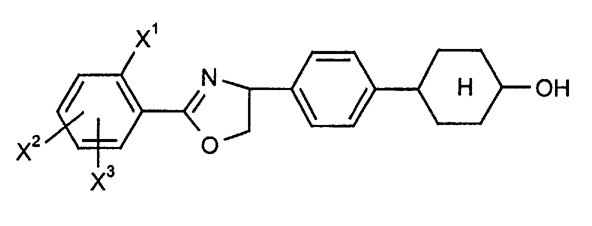 in welcher
   X¹, X² und X³ die oben angegebene Bedeutung haben,
   mit Halogeniden der Formel (VIII)

   R¹¹-Hal (VIII)

   in welcher
   - R¹¹: für Alkyl oder gegebenenfalls substituiertes Benzyl steht und
   - Hal: für Halogen steht
   in Gegenwart einer Base und gegebenenfalls in Gegenwart eines Verdünnungsmittels umsetzt.

Weiter wurde gefunden, daß die neuen 4-Cyclohexylphenyl-oxazoline der Formel (I) sehr gut zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- X¹: steht besonders bevorzugt für Fluor, Chlor oder Methyl (insbesondere für Fluor oder Chlor, hervorgehoben für Fluor).
- X² und X³: sind gleich oder verschieden und stehen besonders bevorzugt für Wasserstoff, für Fluor, Chlor, Methyl, Ethyl, Methoxy, Methylthio, Trifluormethyl, Trifluormethoxy, Amino, Dimethylamino oder Diethylamino (wobei X³ insbesondere für Wasserstoff steht und X² insbesondere für Fluor oder Chlor, hervorgehoben für Fluor, jeweils bevorzugt in der 6-Position).
- R¹: steht besonders bevorzugt für Wasserstoff, -OR³ oder -SR³ und
- R²: steht besonders bevorzugt für den Rest -OR⁴,
oder
- R¹ und R²: stehen gemeinsam besonders bevorzugt für Sauerstoff, für =N-OR⁵ oder =N-NR⁶R⁷.
R³ und R⁴ sind gleich oder verschieden und stehen besonders bevorzugt für Wasserstoff, Methyl, Ethyl, n- oder i-Propyl, n-, i-, s- oder t-Butyl; oder für gegebenenfalls, vorzugsweise im Phenylteil, einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl,
oder gemeinsam mit den Heteroatomen, an die sie gebunden sind, und dem diese verbindenden C-Atom für einen gegebenenfalls einfach bis dreifach, bevorzugt einfach oder zweifach, gleich oder verschieden substituierten 5- oder 6-gliedrigen Ring (der bevorzugt keine weiteren Heteroatome enthält), wobei als Substituenten
gegebenenfalls durch Fluor, Chlor, Methoxy, Ethoxy, n- oder i-Propoxy, n-Butyloxy, Allyloxy, Benzyloxy (welches jeweils im Ring gegebenenfalls einfach bis dreifach, bevorzugt einfach oder zweifach, gleich oder verschieden durch Fluor, Chlor, Brom, Methyl, Ethyl, Methoxy, Ethoxy oder Phenoxy substituiert ist) oder Phenoxy substituiertes C₁-C₁₈-Alkyl, C₂-C₄-Alkenyl, gegebenenfalls einfach bis dreifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl, Ethoxycarbonyl, Phenyl, Halogenphenyl, Benzyloxy oder Halogenbenzyloxy substituiertes Phenyl oder -NR¹²R¹³ in Frage kommen.
R¹² steht besonders bevorzugt für Wasserstoff, Methyl, Ethyl, n-oder i-Propyl, n-, i-, s- oder t-Butyl, Allyl, Propargyl, Trifluormethyl oder für gegebenenfalls, vorzugsweise im Phenylteil, einfach bis dreifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl.
R¹³ steht besonders bevorzugt für Methyl, Ethyl, n- oder i-Propyl, n-, i-, s- oder t-Butyl, Methylcarbonyl, Ethylcarbonyl, Methoxycarbonyl, Ethoxycarbonyl, für jeweils gegebenenfalls, vorzugsweise im Phenylteil, einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl, für -CONR¹⁴R¹⁵ oder -SO₂NR¹⁴R¹⁵.
R¹⁴ steht besonders bevorzugt für Wasserstoff, Methyl, Ethyl, n-oder i-Propyl, n-, i-, s- oder t-Butyl, Allyl, Propargyl, Trifluormethyl oder für gegebenenfalls, vorzugsweise im Phenylteil, einfach oder zweifach, gleich oder verschieden durch Fluor, Chlor, Brom, Methyl, Ethyl, n- oder i-Propyl, n-, i-, s-oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl.
R¹⁵ steht besonders bevorzugt für Methyl, Ethyl, n- oder i-Propyl, n-, i-, s- oder t-Butyl, Methylcarbonyl, Ethylcarbonyl, Methoxycarbonyl, Ethoxycarbonyl, für jeweils gegebenenfalls, vorzugsweise im Phenylteil einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl.
- R⁵: steht besonders bevorzugt für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil einfach oder zweifach, gleich oder verschieden durch Fluor, Chlor, Brom, Methyl, Ethyl, n-oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl.
- R⁶: steht besonders bevorzugt für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil einfach oder zweifach, gleich oder verschieden durch Fluor, Chlor, Brom, Methyl, Ethyl, n-oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl.
- R⁷: steht besonders bevorzugt für Methyl, Ethyl, n- oder i-Propyl, n-, i-, s-oder t-Butyl, Methylcarbonyl, Ethylcarbonyl, Methoxycarbonyl, Ethoxycarbonyl, für jeweils gegebenenfalls, vorzugsweise im Phenylteil einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl oder für -CONR⁸R⁹ oder -SO₂NR⁸R⁹.
- R⁸: steht besonders bevorzugt für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil einfach bis dreifach, gleich oder verschieden durch Fluor, Chlor, Brom, Methyl, Ethyl, n-oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl.
- R⁹: steht besonders bevorzugt für Methyl, Ethyl, n- oder i-Propyl, n-, i-, s- oder t-Butyl, Methylcarbonyl, Ethylcarbonyl, Methoxycarbonyl, Ethoxycarbonyl, für jeweils gegebenenfalls, vorzugsweise im Phenylteil einfach bis zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl.

Die oben aufgeführten allgemeinen oder in Vorzugsbereichen aufgeführten Restedefinitionen bzw. Erläuterungen gelten für die Endprodukte und für die Ausgangsund Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der allgemeinen Formel (I), in welchen eine Kombination der vorstehend als bevorzugt (vorzugsweise) aufgeführten Bedeutungen vorliegt.

Erfindungsgemäß besonders bevorzugt werden die Verbindungen der allgemeinen Formel (I), in welchen eine Kombination der vorstehend als besonders bevorzugt aufgeführten Bedeutungen vorliegt.

In den oben und nachstehend aufgeführten Restedefinitionen sind Kohlenwasserstoffreste, wie Alkyl oder Alkenyl - auch in Verbindung mit Heteroatomen wie Alkoxy oder Alkylthio - soweit möglich jeweils geradkettig oder verzweigt.

Bevorzugte erfindungsgemäße Verbindungen sind Stoffe der Formeln (IA) bis (IE) 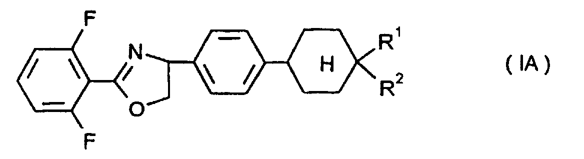 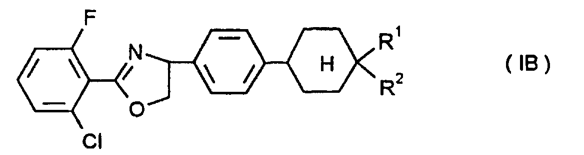 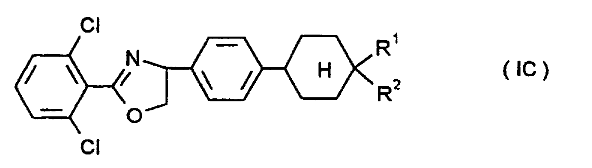 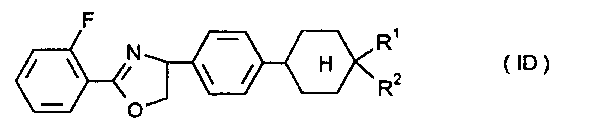 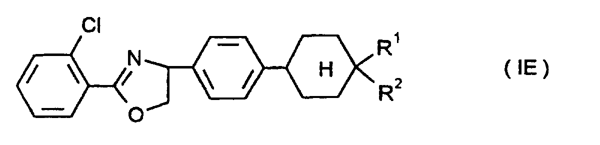 in welchen
- R¹ und R²: die oben genannten Bedeutungen haben.

Bevorzugte erfindungsgemäße Verbindungen sind auch Stoffe der Formel (IF) 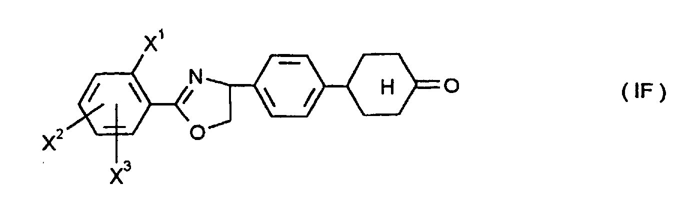 in welcher
- X¹, X² und X³: die obengenannten Bedeutungen haben.

Bevorzugte erfindungsgemäße Verbindungen sind weiterhin Stoffe der Formel (I), d.h. auch (IF), in denen
- X¹: für Fluor, Chlor, C₁-C₂-Alkyl, C₁-C₂-Alkoxy oder C₁-C₂-Alkylthio steht (insbesondere für Fluor),
- X²: für Wasserstoff, Fluor oder Chlor steht (insbesondere für Fluor oder Chlor, hervorgehoben für Fluor, speziell 6-Fluor),
- X³: für Wasserstoff, Fluor, Chlor oder C₁-C₂-Alkyl steht (insbesondere für Wasserstoff),
- R¹: für den Rest -OR³ steht und
- R²: für den Rest -OR⁴ steht, wobei
- R³ und R⁴: gemeinsam mit den O-Atomen, an die sie gebunden sind und dem diese verknüpfenden C-Atom für einen fünfgliedrigen Ring (der bevorzugt keine weiteren Heteroatome enthält) stehen, der gegebenenfalls substituiert sein kann durch C₁-C₁₈-Alkyl oder durch gegebenenfalls einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy oder C₁-C₄-Halogenalkylthio substituiertes Phenyl.

Verwendet man zur Durchführung des erfindungsgemäßen Verfahrens (a) beispielsweise 2-(2,6-Difluorbenzoylamido-2-[4-(4-oxo-cyclohexyl)-phenyl]-1-chlorethan als Ausgangsstoff, so kann der Reaktionsablauf durch das folgende Formelschema wiedergegeben werden:

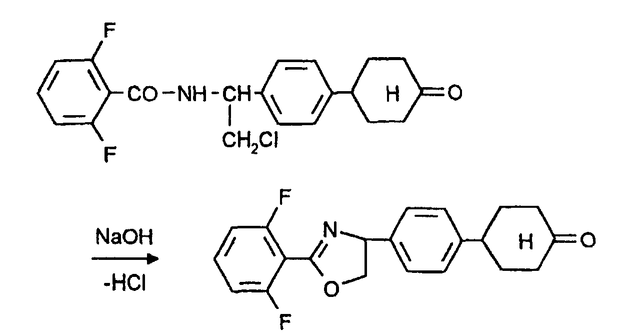

Verwendet man zur Durchführung des erfindungsgemäßen Verfahrens (b) gemäß Variante (b1) beispielsweise 2-(2,6-Difluorphenyl-4-[4-(4-oxo-cyclohexyl)-phenyl]-1,3-oxazolin und (4-Chlorphenyl)-ethan-1,2-diol als Ausgangsstoffe, so kann der Reaktionsablauf durch das folgende Formelschema wiedergegeben werden:

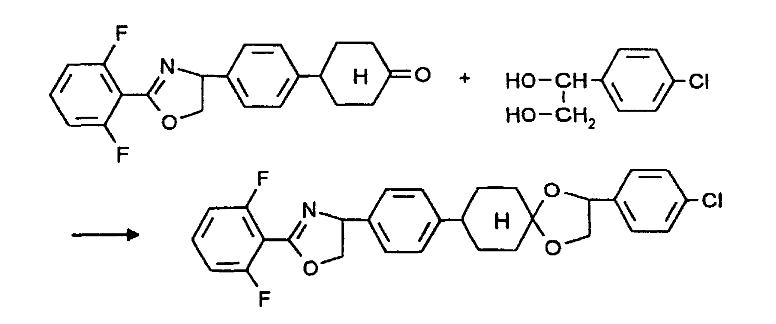

Verwendet man zur Durchführung des erfindungsgemäßen Verfahrens (b) gemäß Variante (b2) beispielsweise 2-(2,6-Difluorphenyl)-4-[4-(4-oxo-cyclohexyl)-phenyl]-1,3-oxazolin und Hydroxylamin-hydrochlorid als Ausgangsstoffe, so kann der Reaktionsablauf durch das folgende Formelschema wiedergegeben werden:

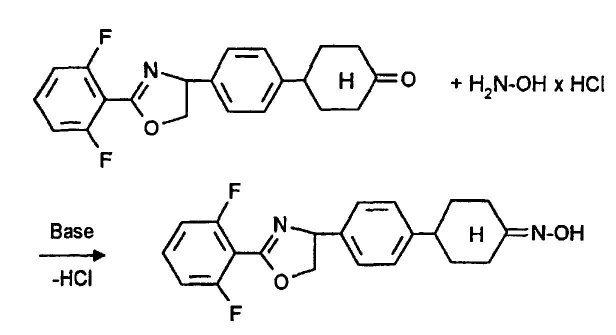

Verwendet man zur Durchführung des erfindungsgemäßen Verfahrens (b) gemäß Variante (b3) beispielsweise 2-(2,6-Difluorphenyl)-4-[4-(4-oxo-cyclohexyl)-phenyl]-1,3-oxazolin und Methylhydrazin als Ausgangsstoffe, so kann der Reaktionsablauf durch das folgende Formelschema wiedergegeben werden:

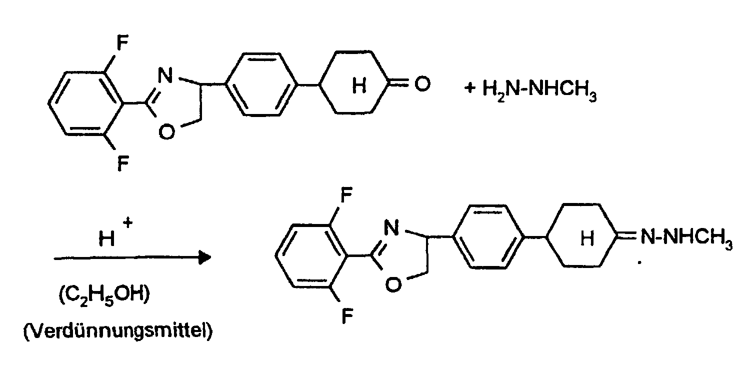

Verwendet man zur Durchführung des erfindungsgemäßen Verfahrens (c) beispielsweise 2-(2,6-Difluorphenyl)-4-[4-(4-hydroxycyclohexyl)-phenyl]-1,3-oxazolin und Benzylchlorid als Ausgangsstoffe, so kann der Reaktionsablauf durch das folgende Formelschema wiedergegeben werden:

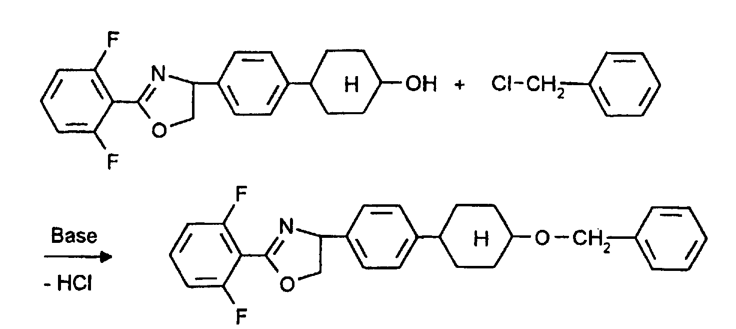

Die beim erfindungsgemäßen Verfahren (a) als Ausgangsstoffe zu verwendenden Chlorethan-Derivate der Formel (II) sind neu.

Sie werden beispielsweise erhalten, indem man Amid-Derivate der Formel (IX) 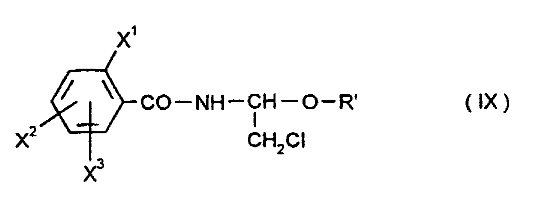 in welcher
- X¹, X² und X³: die oben angegebene Bedeutung haben und
- R': für C₁-C₄-Alkyl, vorzugsweise Methyl oder Ethyl steht,
mit Phenyl-Derivaten der Formel (X) 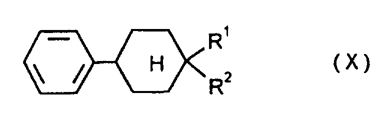 in welcher
- R¹ und R²: die oben angegebene Bedeutung haben,
in Gegenwart eines sauren Katalysators und in Gegenwart eines Verdünnungsmittels umsetzt.

Als Verdünnungsmittel kommen alle gegenüber den Reaktionsteilnehmern inerten Lösungsmittel in Frage.

Vorzugsweise verwendet werden Kohlenwasserstoffe, wie Hexan, Halogenkohlenwasserstoffe, wie Methylenchlorid, Chloroform, Chlorbenzol, o-Dichlorbenzol; sowie Schwefelkohlenstoff..

Als saurer Katalysator kommen prinzipiell alle anorganischen oder organischen Säuren oder Lewis-Säuren in Frage. Bevorzugt verwendet werden beispielsweise Schwefelsäure, Methansulfonsäure, Benzolsulfonsäure, wasserfreie Flußsäure, Aluminiumchlorid, Titantetrachlorid, Phosphoroxychlorid, Bortrifluorid-Etherat. Ein Überschuß an Säure kann gegebenenfalls auch als Verdünnungsmittel dienen.

Die Reaktionstemperatur kann innerhalb eines größeren Bereichs variiert werden. Im allgemeinen arbeitet man bei Temperaturen zwischen -20°C und 150°C, bevorzugt zwischen -20°C und +20°C.

Die Umsetzung wird im allgemeinen unter Normaldruck durchgeführt.

Die Verbindungen der Formeln (IX) und (X) werden im allgemeinen in äuqimolaren Mengen eingesetzt; es ist jedoch auch möglich, einen Überschuß der einen oder anderen Verbindung zu verwenden.

Die Amid-Derivate der Formel (IX) sind bekannt (vgl. z.B. EP-A 0 594 179) und/oder können nach dort angegebenen Methoden erhalten werden.

Die Phenyl-Derivate der Formel (X) sind allgemein bekannte Verbindungen der organischen Chemie und/oder können in allgemein bekannter Art und Weise erhalten werden.

Die bei den erfindungsgemäßen Verfahren (b) bzw. (c) als Ausgangsstoffe zu verwendenden Verbindungen der Formel (Ia) bzw. (Ib) sind erfindungsgemäße Verbindungen. Die Verbindungen der Formel (Ib) können auch erhalten werden, wenn man die Carbonylgruppe in den Verbindungen der Formel (Ia) in üblicher Art und Weise reduziert.

Die beim erfindungsgemäßen Verfahren (b) außerdem als Ausgangsstoffe zu verwendenden Alkohole der Formel (III), Thioalkohole der Formel (IV), Verbindungen der Formel (V), Hydroxylamine der Formel (VI) und Hydrazine der Formel (VII) sind allgemein bekannte Verbindungen der Organischen Chemie und/oder können in allgemein bekannter Art und Weise erhalten werden.

In der Formel (V) steht A vorzugsweise für eine gegebenenfalls einfach oder zweifach, gleich oder verschieden substituierte C₂- oder C₃-Kohlenwasserstoffkette steht, wobei als Substituenten diejenigen in Frage kommen, die oben für R³ und R⁴ in ihrer Bedeutung als gemeinsamer Ring als Substituenten genannt wurden.

Die beim erfindungsgemäßen Verfahren (c) außerdem als Ausgangsstoffe zu verwendenden Halogenide der Formel (VIII) sind allgemein bekannte Verbindungen der Organischen Chemie.

Hal steht in der Formel (VIII) vorzugsweise für Chlor, Brom oder Jod.

R¹¹ in der Formel (VIII) steht für Alkyl oder gegebenenfalls substituiertes Benzyl, jeweils wie für R⁴ definiert.

Als Verdünnungsmittel kommen beim erfindungsgemäßen Verfahren (a) alle inerten organischen Lösungsmittel in Frage. Sie können gegebenenfalls in Mischung mit Wasser verwendet werden. Bevorzugt verwendet werden Kohlenwasserstoffe wie Toluol, Xylol, Tetralin, Hexan, Cyclohexan, Halogenkohlenwasserstoffe wie Methylenchlorid, Chloroform, Chlorbenzol, o-Dichlorbenzol, Alkohole wie Methanol, Ethanol, Glykol, die isomeren Propanole, Butanole, Pentanole, Ether wie Diethylether, Diisopropylether, Dimethoxyethan, Tetrahydrofuran, Dioxan, Nitrile wie Acetonitril oder Butyronitril, Amide wie Dimethylformamid, Sulfoxide wie Dimethylsulfoxid, ferner Sulfolan. Besonders bevorzugt werden Alkohole oder Amide verwendet.

Als Base kommen beim erfindungsgemäßen Verfahren (a) alle üblichen Säureakzeptoren in Frage.

Vorzugsweise verwendbar sind tertiäre Amine wie Triethylamin, Pyridin, Diazabicyclooctan (DABCO), Diazabicycloundecen (DBU), Diazabicyclononen (DBN), N,N-Dimethylanilin, ferner Erdalkalimetalloxide wie Magnesium- und Calciumoxid, außerdem Alkali- und Erdalkalimetallcarbonate wie Natriumcarbonat, Kaliumcarbonat und Calciumcarbonat, Alkalihydroxide wie Natrium- und Kaliumhydroxid, ferner Alkoholate wie Natriumethanolat oder Kalium-tert.-butylat. Besonders bevorzugt werden Alkalihydroxide und Alkalialkoholate.

Gegebenenfalls wird beim erfindungsgemäßen Verfahren (a) in Gegenwart eines Phasentransferkatalysators gearbeitet. Als Phasentransferkatalysator kommen beispielsweise Ammoniumverbindungen wie Tetraoctylammoniumbromid oder Benzyltriethylammoniumchlorid in Frage.

Die Reaktionstemperatur kann beim erfindungsgemäßen Verfahren (a) in einem größeren Bereich variiert werden. Im allgemeinen arbeitet man bei Temperaturen zwischen -10°C und 150°C, bevorzugt zwischen 0°C und 100°C.

Die Umsetzung wird im allgemeinen unter Normaldruck durchgeführt.

Im allgemeinen wird eine äquimolare Menge an Base eingesetzt. Es ist aber auch möglich, mit einem Basenüberschuß zu arbeiten.

Es wird in üblicher Weise aufgearbeitet.

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, gegebenenfalls deren Gemische mit Wasser oder reines Wasser.

Das erfindungsgemäße Verfahren (b) gemäß Variante (b1) wird gegebenenfalls in Gegenwart eines Katalysators durchgeführt. Hierzu gehören beispielsweise Säuren, z.B. Toluolsulfonsäure, Salze von Alkalihydroxiden mit Säuren, Salze von Aminen (insbesondere von Pyridin mit anorganischen Säuren, beispielsweise Pyridinhydrochlorid).

Das erfindungsgemäße Verfahren (b) gemäß Variante (b2) wird gegebenenfalls in Gegenwart einer geeigneten Säure oder Base (als wasserabspaltendes Mittel) durchgeführt. Als solche kommen z.B. p-Toluolsulfonsäure, Schwefelsäure, Pyridinhydrochlorid oder Natriumacetat in Frage.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und +150°C, vorzugsweise bei Temperaturen zwischen 30°C und +120°C (gegebenenfalls mit einem Wasserabscheider).

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol an Verbindung der Formel (Ia) im allgemeinen 1,0 bis 3,0 Mol, vorzugsweise 1,0 bis 2,0 Mol an Verbindungen der Formeln (III), (IV), (V), (VI) oder (VII) ein. Die Reaktionsdurchführung, Aufarbeitung und Isolierung der Reaktionsprodukte erfolgt nach bekannten Verfahren.

Als Verdünnungsmittel kommen bei der Durchführung des erfindungsgemäßen Verfahrens (c) alle üblichen inerten, organischen Solventien in Betracht. Vorzugsweise verwendbar sind aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tbutylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Als Basen kommen bei der Durchführung des erfindungsgemäßen Verfahrens (c) alle üblichen anorganischen und organischen Basen in Frage. Vorzugsweise verwendbar sind Alkalihydroxide (wie Natriumhydroxid oder Kaliumhydroxid), Alkalialkoholate (wie Natriummethanolat oder Natriumethanolat), Butyllithium oder Natriumhydrid.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +180°C, vorzugsweise bei Temperaturen zwischen 0°C und 130°C.

Das erfindungsgemäße Verfahren (c) wird üblicherweise unter Normaldruck durchgeführt. Es ist jedoch auch möglich unter erhöhtem oder vermindertem Druck zu arbeiten.

Bei der Durchführung des erfindungsgemäßen Verfahrens (c) setzt man auf 1 Mol an Verbindung der Formel (Ib) im allgemeinen 1,0 bis 5,0 Mol, vorzugsweise 1,0 bis 3,0 Mol an Halogenid der Formel (VIII) ein. Die Durchführung der Reaktion und die Aufarbeitung erfolgen nach üblichen Methoden.

Erfindungsgemäß ist auch ein Verfahren zur Bekämpfung von Schädlingen, bei den Verbindungen der Formel (I), wie vorstehend definiert, auf Schädlinge und/oder den Lebensraum einwirken lässt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch hervorragende insektizide und akarizide Wirksamkeit aus.

Sie lassen sich mit besonders gutem Erfolg zur Bekämpfung von pflanzenschädigenden Insekten, wie beispielsweise gegen die Meerrettichblattkäfer-Larven (Phaedon cochleariae), die grüne Reiszikade (Nephotettix cinctriceps), die Raupen des Eulenfalters (Spodoptera frugiperda) und die Pfirsichblattläuse (Mycus persicae) oder zur Bekämpfung von pflanzenschädigenden Milben, wie beispielsweise gegen die gemeine Spinnmilbe (Tetranychus urticae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 % und bevorzugt daneben Streckmittel und/oder oberflächenaktive Mittel.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3 -methoxyacrylat; Methyl-(E)-methoximino[alpha-(otolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301/5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine gute entwicklungshemmende Wirkung gegen Fliegenlarven von Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze
   wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

### Herstellungsbeispiele

### Beispiel 1

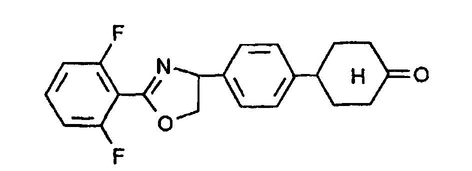

### (Verfahren a)

Zu 20,8 g (0,0531 Mol) 2-(2,6-Difluorbenzoylamido)-2-[4-(4-oxo-cyclohexyl)-phenyl]-1-chlorethan in 130 ml absolutem Dimethylformamid tropft man bei 5°C 3,5 ml (0,05841 Mol) 45 %-ige Natronlauge. Man läßt das Reaktionsgemisch auf Raumtemperatur erwärmen und rührt über Nacht. Anschließend wird das Lösungsmittel im Wasserstrahlvakuum abdestilliert, der Rückstand in 300 ml Essigester aufgenommen, mehrfach mit Wasser gewaschen, über Natriumsulfat getrocknet, filtriert, eingeengt und säulenchromatographisch (Kieselgel, Toluol/Essigester 9/1) gereinigt.

Man erhält 16,5 g (79,6 % der Theorie) 2-(2,6-Difluorphenyl)-4-[4-(4-oxo-cyclohexyl)-phenyl)-1,3-oxazolin mit einem logp^{*)} = 2.28.
*) logp: Dekadischer Logarithmus des n-Octanol/Wasser-Verteilungskoeffizienten, bestimmt durch HPLC-Analytik an reversed phase mit H₂O/CH₃CN.

### Herstellung des Ausgangsproduktes

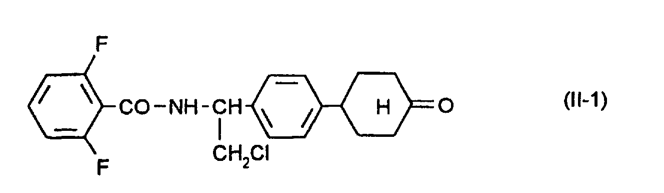

17,4 g (0,1 Mol) 4-Phenylcyclohexanon und 24,3 g (0,1 Mol) N-(2-Chlor-1-methoxyethyl)-2,6-difluorbenzoesäureamid in 100 ml absolutem Methylenchlorid werden bei 5°C portionsweise mit 48,7 g (0,3 Mol) wasserfreiem Eisen-III-chlorid versetzt. Man läßt das Reaktionsgemisch innerhalb von 4 Stunden auf Raumtemperatur erwärmen und rührt bei dieser Temperatur 16 Stunden.

Danach wird das Reaktionsgemisch in 1 Liter Eiswasser eingerührt, die organische Phase abgetrennt, über Natriumsulfat getrocknet, filtriert, im Wasserstrahlvakuum eingeengt und säulenchromatographisch (Kieselgel, Methylenchlorid/Essigester) gereinigt.

Man erhält 10,4 g (26,6 % der Theorie) 2-(2,6-Difluorbenzoylamido)-2-[4-(4-oxocyclohexyl)-phenyl]-chlorethan mit einem logp^{*)} = 2.54. bestimmt durch HPLC-Analytik an reversed phase mit H₂O/CH₃CN.
^{*)} logp: Dekadischer Logarithmus des n-Octanol/Wasser-Verteilungskoeffizienten,

### Beispiel 2

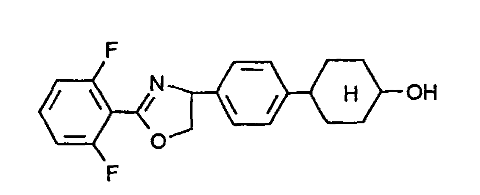

### (Verfahren a)

Zu 5 g (0,01296 Mol) 2-(2,6-Difluorbenzoylamido)-2-[4-(4-hydroxicyclohexyl)-phenyl]-1-chlorethan in 50 ml Dimethylformamid tropft man bei 5°C 0,8 ml (0,01296 Mol) 45 %-ige Natronlauge zu. Man läßt das Reaktionsgemisch auf Raumtemperatur erwärmen, rührt über Nacht und gibt es anschließend in 150 ml Eiswasser. Danach wird mit Dichlormethan extrahiert, über Natriumsulfat getrocknet, filtriert, eingeengt und säulenchromatographisch (Kieselgel, Toluol/Essigester 1/1) gereinigt.

Man erhält 3,4 g (73,5 % der Theorie) 2-(2,6-Difluorphenyl)-4-[4-(4-hydroxicyclohexyl)phenyl]-1,3-oxazolin als reines Diastereomeres mit einem logp^{*)} = 2.53.
^{*)} logp: Dekadischer Logarithmus des n-Octanol/Wasser-Verteilungskoeffizienten, bestimmt durch HPLC-Analytik an reversed phase mit H₂O/CH₃CN.

### Beispiel 3

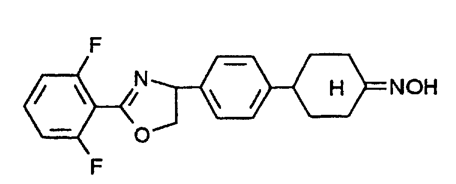

### (Verfahren b, Variante b2)

Zu 8,8 g (0,0247 Mol) 2-(2,6-Difluorphenyl)-4-[4-(4-oxo-cyclohexyl)-phenyl]-1,3-oxazolin (Beispiel 1) in 100 ml 1,2-Dimethoxyethan gibt man zunächst eine Lösung von 21 g (0,0247 Mol) Natriumacetat in 10 ml Wasser und danach 1,7 g (0,0247 Mol) Hydroxylamin-hydrochlorid. Das Reaktionsgemisch wird 16 Stunden bei Raumtemperatur gerührt.

Nach Abdestillieren des Lösungsmittels wird der Rückstand in 300 ml Essigester aufgenommen, mit 200 ml Wasser gewaschen, über Natriumsulfat getrocknet, filtriert, eingeengt und säulenchromatographisch (Kieselgel, Toluol/Essigester 9/1) gereinigt.

Man erhält 6,7 g (60,9 % der Theorie) 2-(2,6-Difluorphenyl)-4-[4-(4-oximino-cyclohexyl)-phenyl]-1,3-oxazolin mit einem logp^{*)} = 2.57.
^{*)} logp: Dekadischer Logarithmus des n-Octanol/Wasser-Verteilungskoeffizienten, bestimmt durch HPLC-Analytik an reversed phase mit H₂O/CH₃CN.

### Beispiel 4

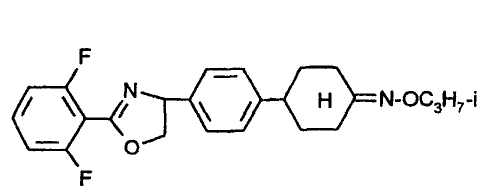

### (Verfahren b, Variante b2)

Zu 0,9 g (0,0024 Mol) 2-(2,6-Difluorphenyl)-4-[4-(4-oximino-cyclohexyl)-phenyl]-1,3-oxazolin (Beispiel 3) in 15 ml absolutem Acetonitril gibt man 0,67 g (0,0048 Mol) Kaliumcarbonat und 0,48 ml (0,0048 Mol) Isopropyliodid.

Nach Zugabe von 2 Tropfen Tetraethoxyethylenammoniumhydroxid läßt man das Reaktionsgemisch über Nacht unter Rückfluß nachrühren und nach weiterer Zugabe von 3 Tropfen Tetrabutylammoniumhydroxid läßt man wiederum unter Rückfluß über Nacht nachrühren. Nach Abdestillieren des Lösungsmittels gibt man auf 100 ml Essigester, wäscht mit 100 ml Wasser, trocknet über Natriumsulfat, filtriert, engt ein und reinigt säulenchromatographisch (Kieselgel, Toluol/Essigester 8/2).

Man erhält 0,2 g (20,2 % der Theorie) 2-(2,6-Difluorphenyl)-4-[4-(4-i-propoximinocyclohexyl)-phenyl]-1,3-oxazolin mit einem logp^{*)}= 4.50.
^{*)} logp: Dekadischer Logarithmus des n-Octanol/Wasser-Verteilungskoeffizienten, bestimmt durch HPLC-Analytik an reversed phase mit H₂O/CH₃CN.

### Beispiel 5

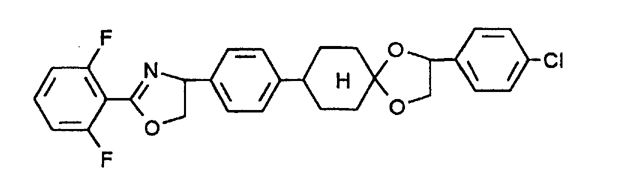

### (Verfahren b, Variante b1)

Zu 1,8 g (0,005 Mol) 2-(2,6-Difluorphenyl)-4-[4-(4-oxo-cyclohexyl)-phenyl]-1,3-oxazolin (Beispiel 1) in 10 ml n-Butanol gibt man 0,9 g (0,005 Mol) (4-Chlorphenyl)-ethan-1,2-diol und 20 ml Toluol. Nach Zugabe von 1 Tropfen Eisessig rührt man das Reaktionsgemisch 3 Stunden unter Rückfluß. Nach Zugabe einer Spatelspitze p-Toluolsulfonsäure rührt man noch eine Stunde. Nach Abdestillieren des Lösungsmittels wird der Rückstand säulenchromatographisch (Kieselgel, Toluol/Essigester 10/0,5) gereinigt.

Man erhält 1,5 g (58,9 % der Theorie) 2-(2,6-Difluorphenyl)-4-[4-(4,4-p-Chlorphenylethylendioxo-cyclohexyl)-phenyl]-1,3-oxazolin mit einem logp^{*)} = 5.37.
^{*)} logp: Dekadischer Logarithmus des n-Octanol/Wasser-Verteilungskoeffizienten, bestimmt durch HPLC-Analytik an reversed phase mit H₂O/CH₃CN.

Analog den Beispielen 1 bis 5 bzw. gemäß den allgemeinen Angaben zur Herstellung wurden die in der nachfolgenden Tabelle 1 aufgeführten Verbindungen der Formel (I) erhalten:

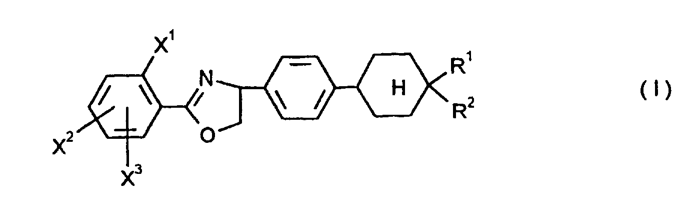

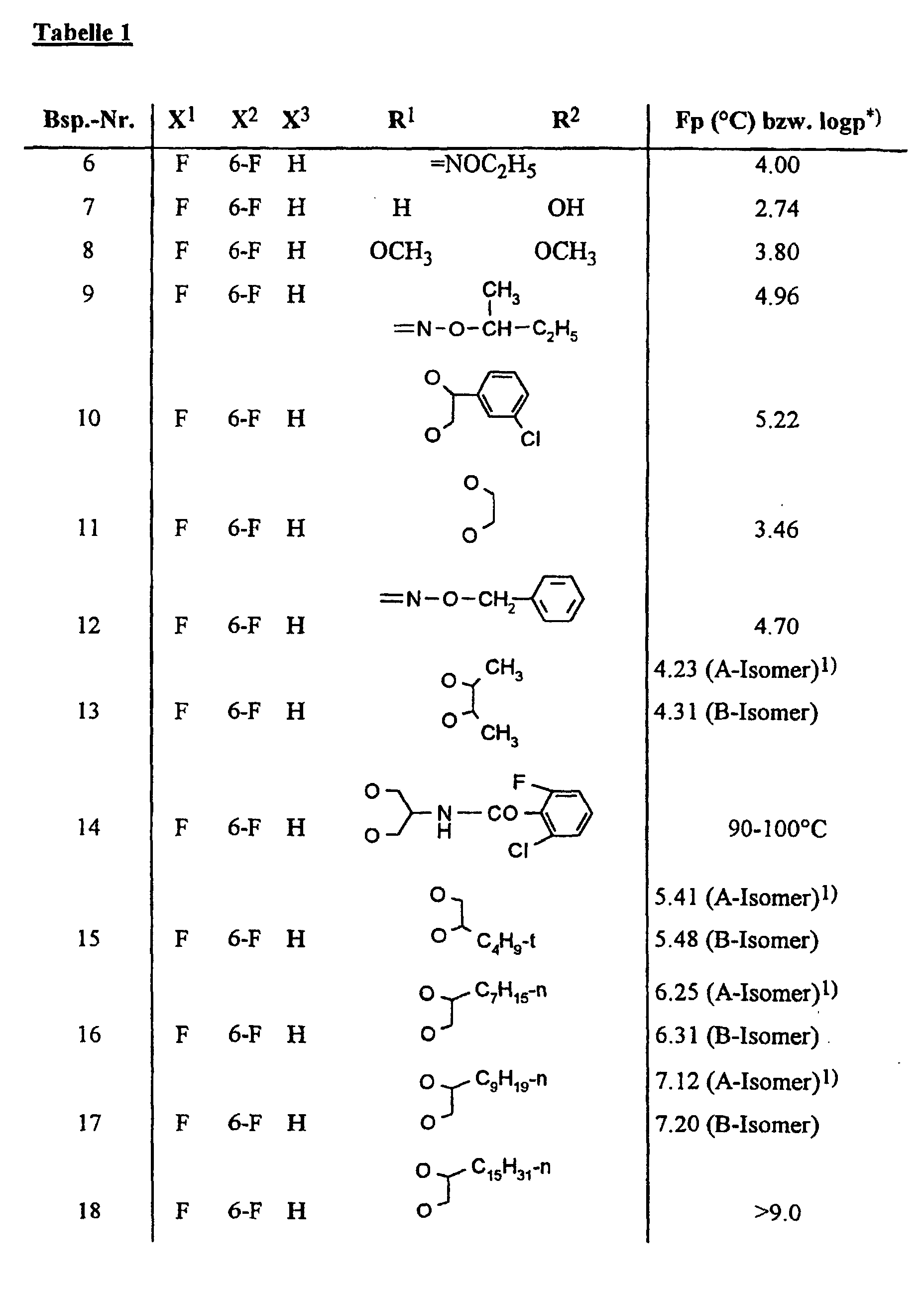

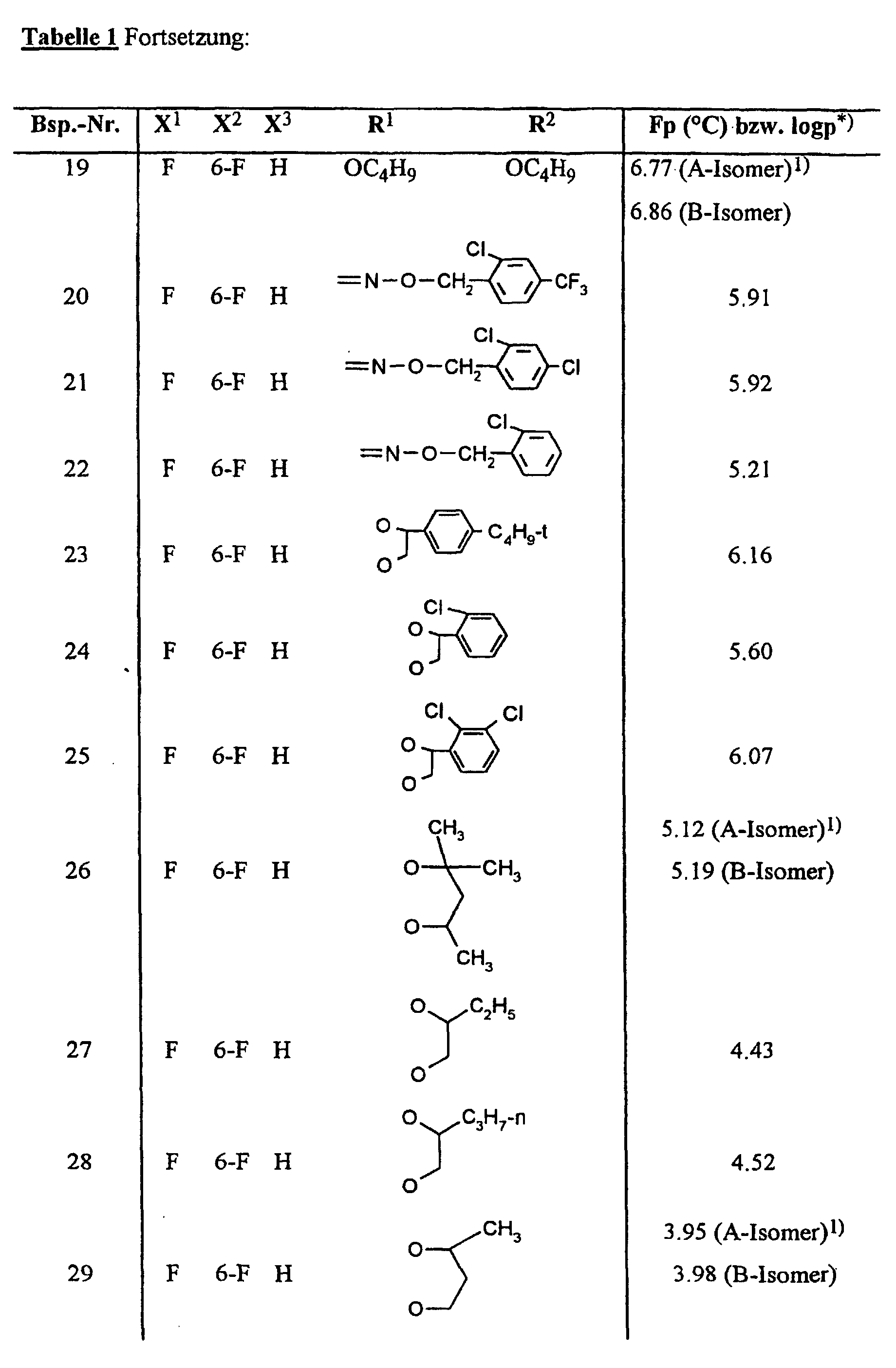

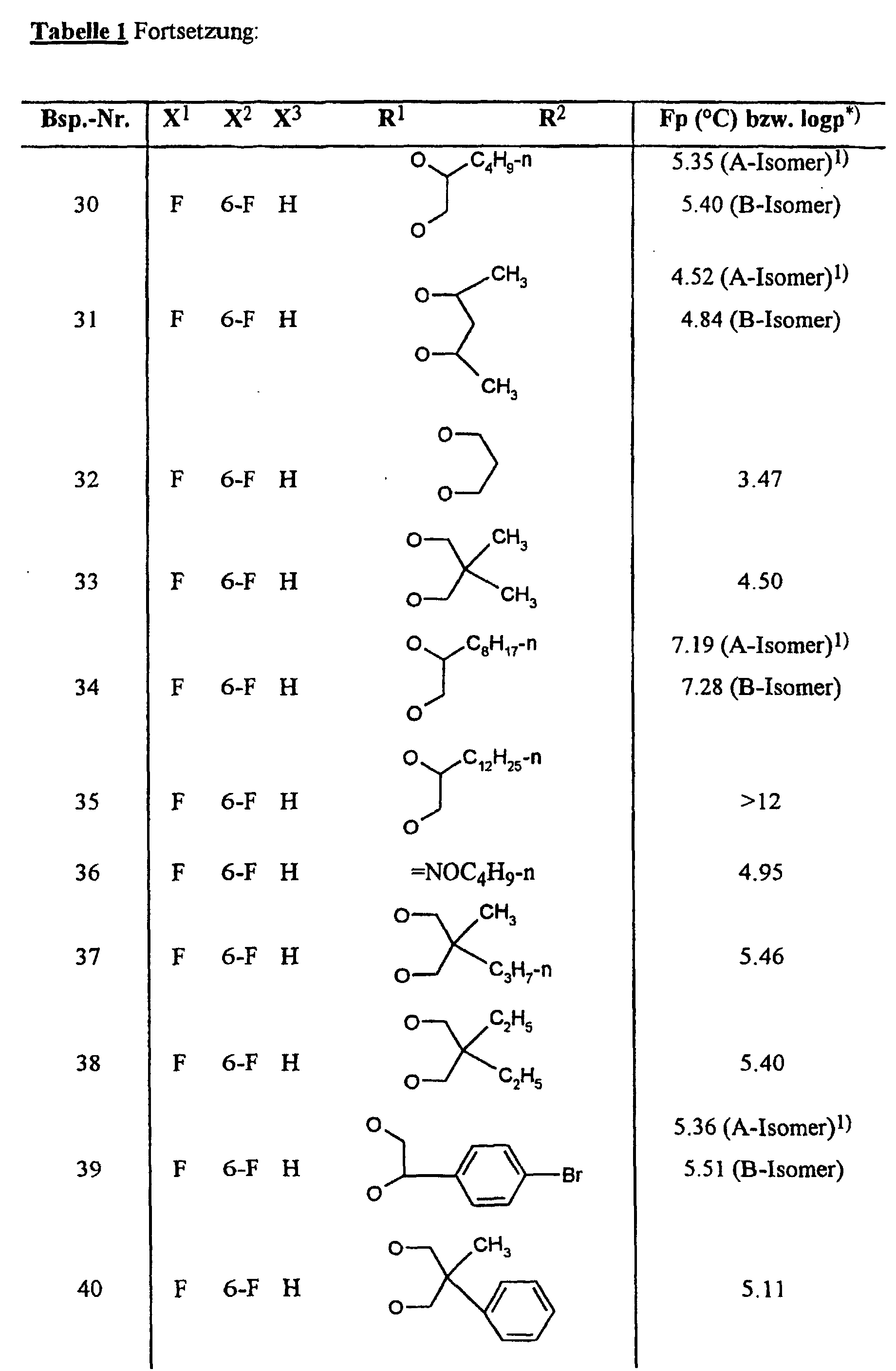

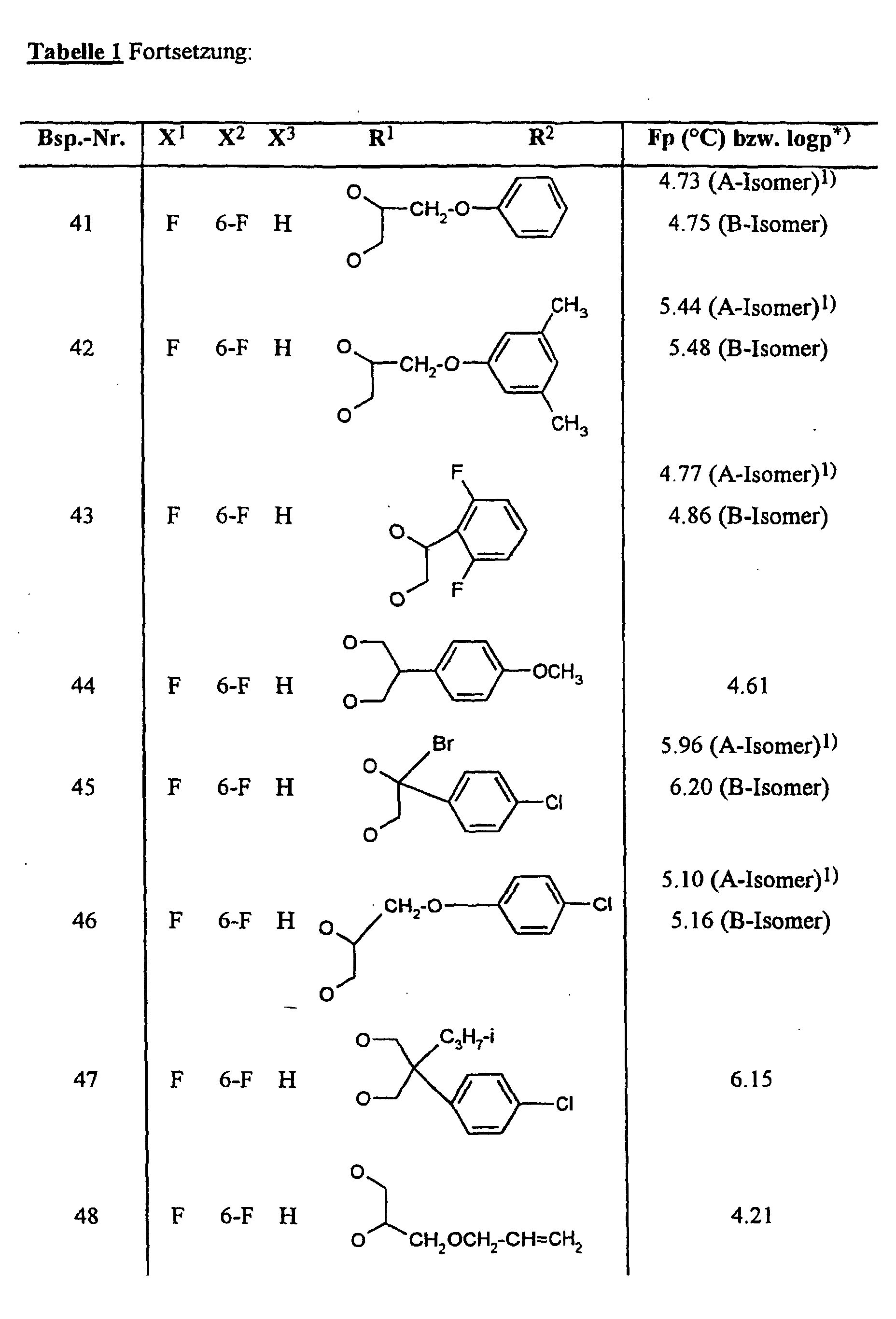

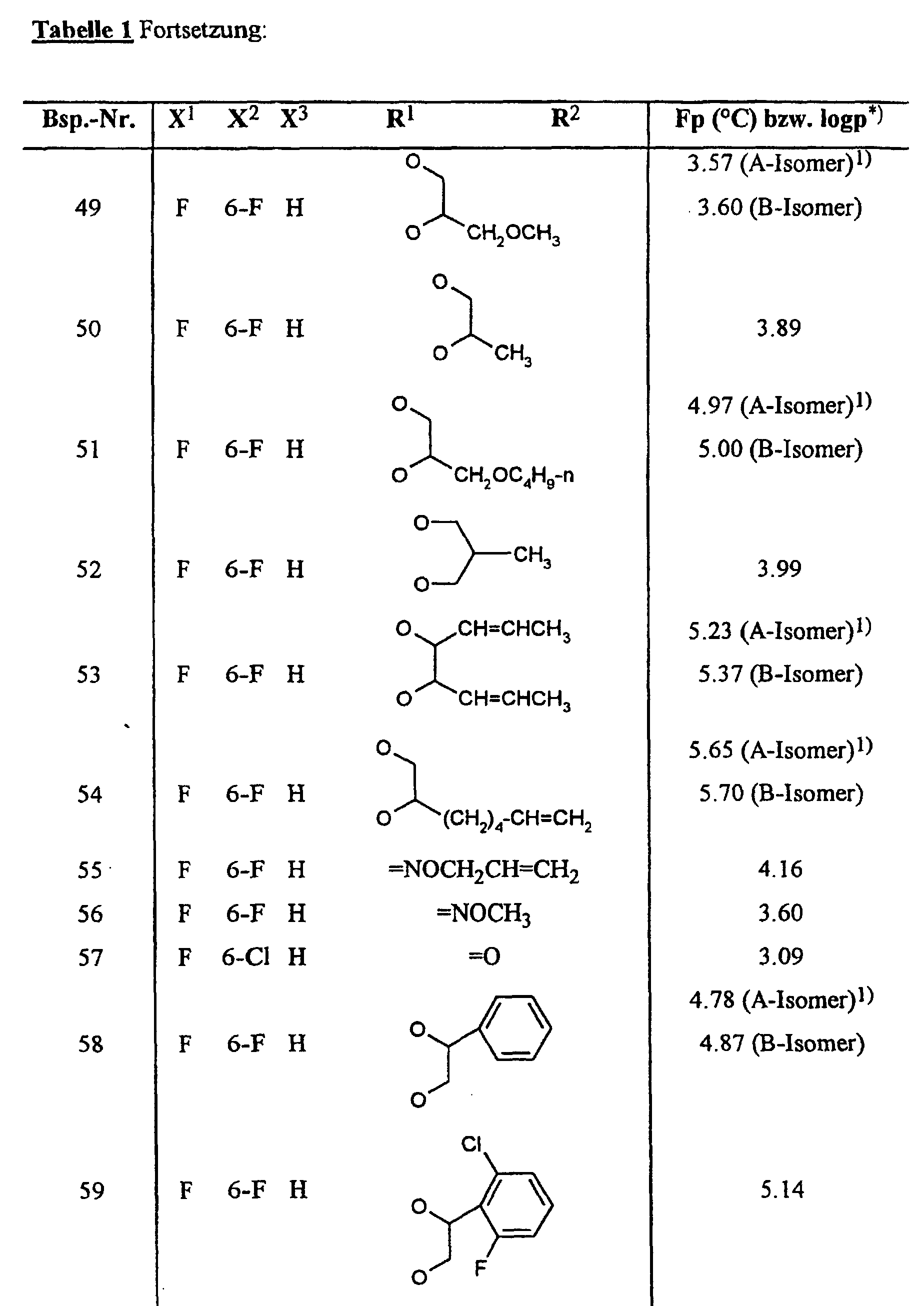

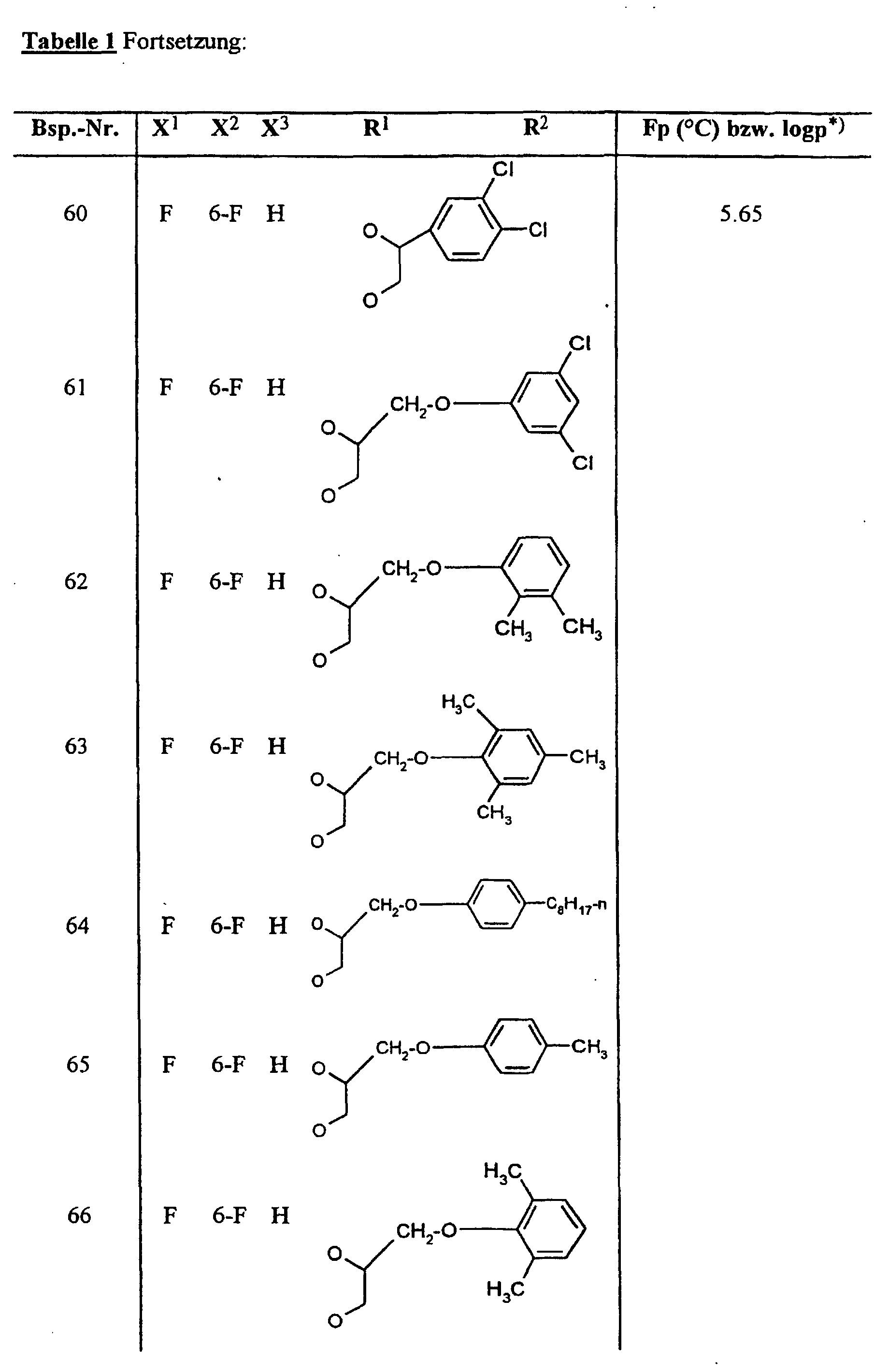

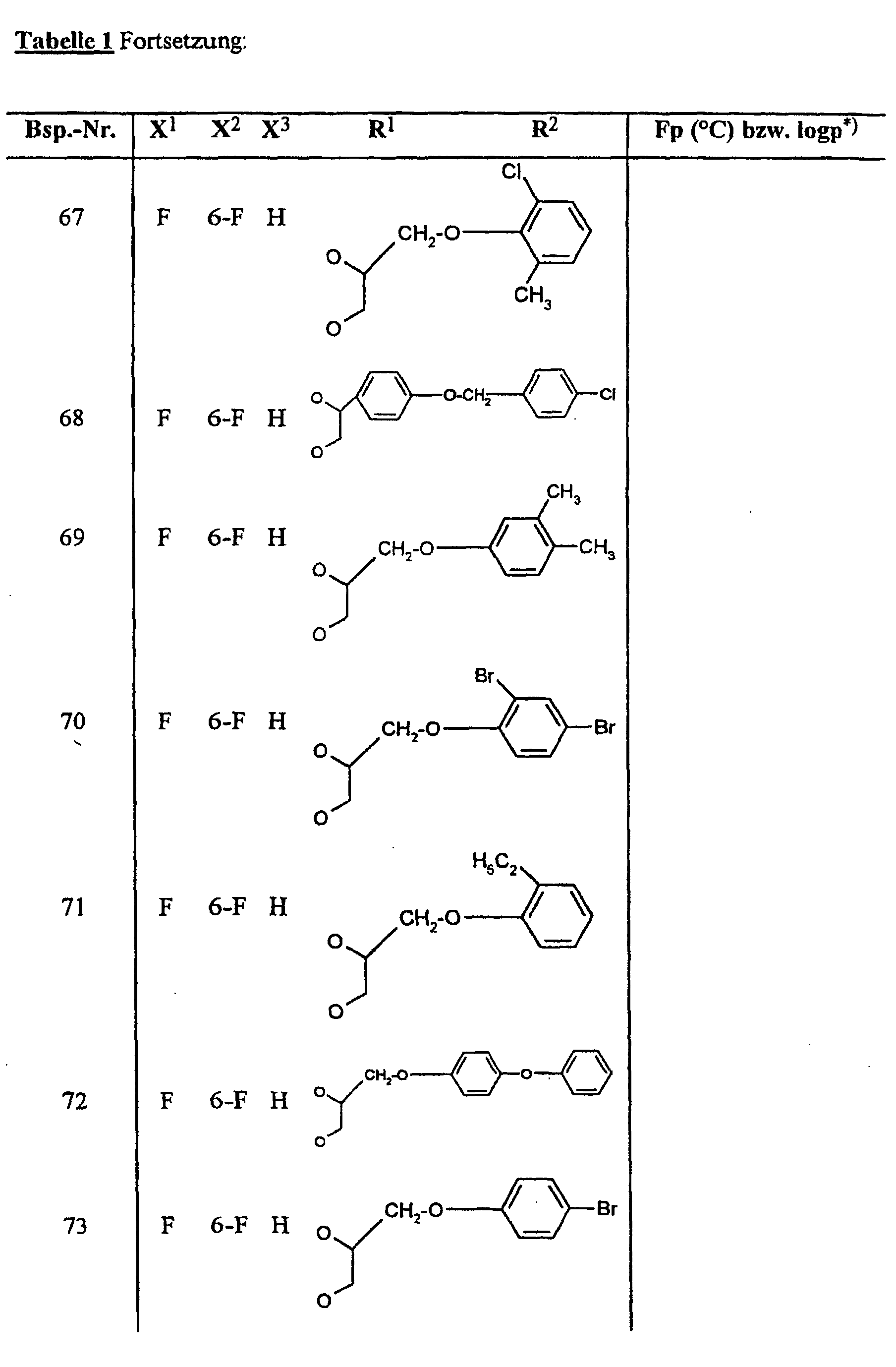

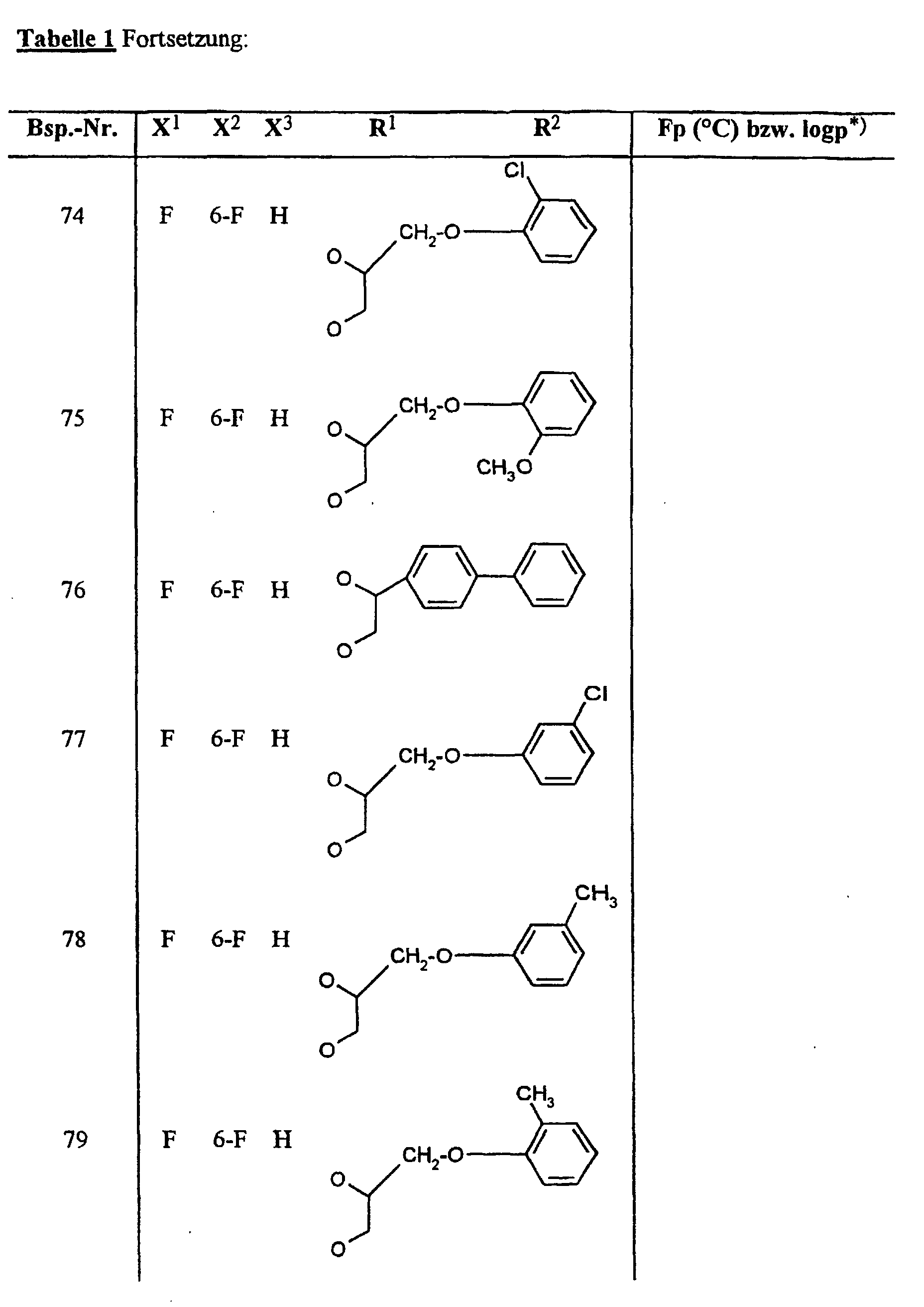

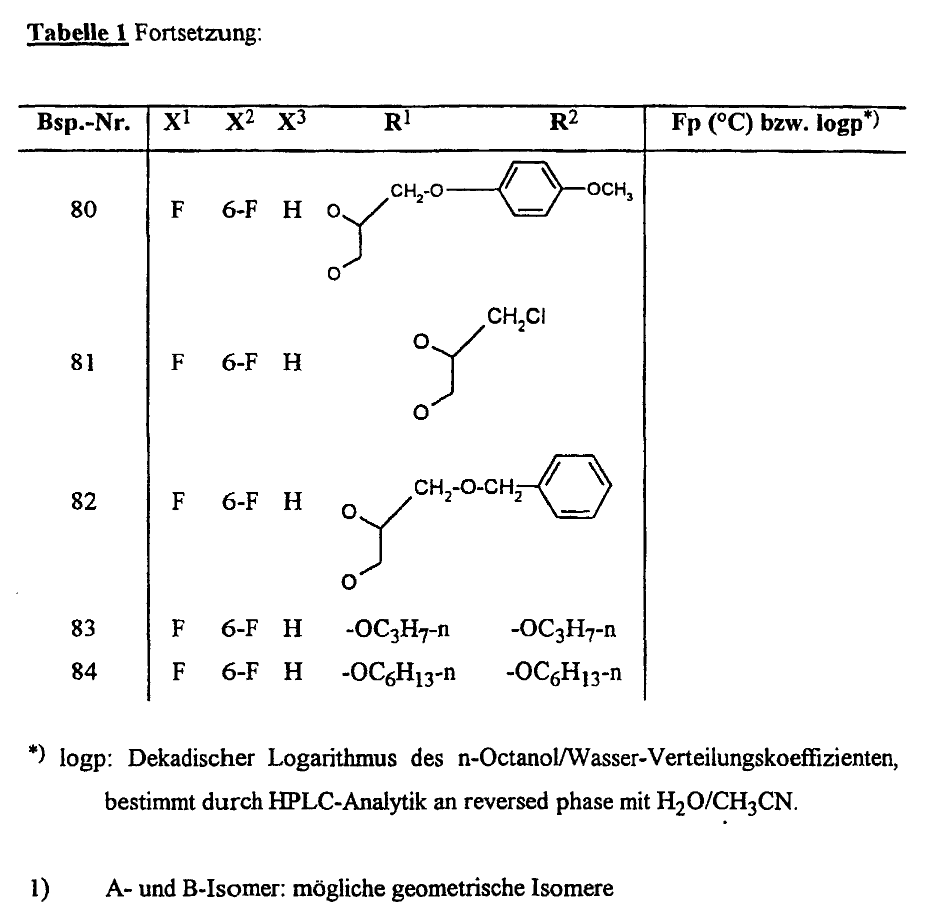

### Anwendungsbeispiele:

In nachfolgenden Anwendungsbeispielen werden nachstehend aufgeführte Verbindungen als Vergleichssubstanzen eingesetzt:

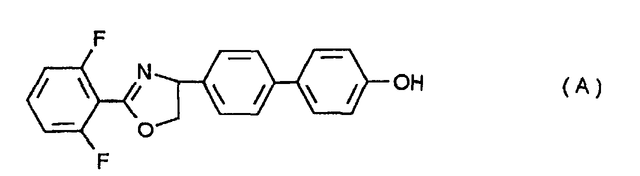

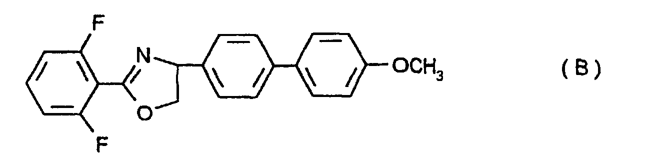

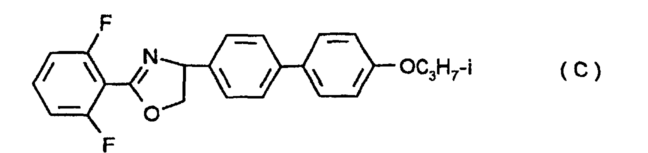

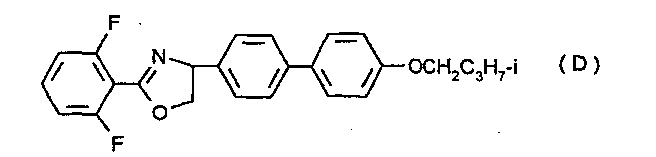

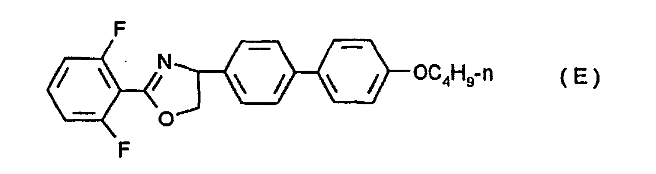

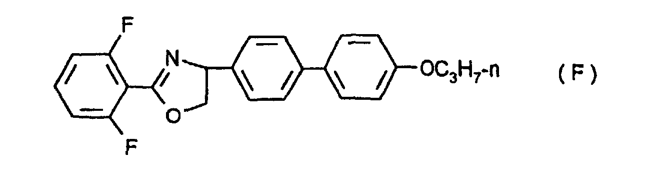

### (Alle Verbindungen bekannt aus EP-A 0 696 584)

### Beispiel A

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test bewirkten bei einer beispielhaften Wirkstoffkonzentration von 0,01 % z.B. die Verbindungen der Herstellungsbeispiele 4 und 8 eine Abtötung von 90 % und der Herstellungsbeispiele 5, 6, 9, 10, 13, 15, 17 und 20 eine Abtötung von 100 %, jeweils nach 7 Tagen, während die bekannten Verbindungen (A) und (B) keine Wirkung zeigten.

### Beispiel B

### Spodoptera frugiperda-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test bewirkten bei einer beispielhaften Wirkstoffkonzentration von 0,1 % z.B. die Verbindungen der Herstellungsbeispiele 4, 5, 6, 9, 10, 17 und 19 eine Abtötung von 100 % jeweils nach 7 Tagen, während die bekannte Verbindung (B) keine Wirkung zeigte.

### Beispiel C

### Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test bewirkten bei einer beispielhaften Wirkstoffkonzentration von 0,1 % z.B. die Verbindungen folgender Herstellungsbeispiele folgende Abtötungen:
1 = 90%; 6 = 100% und 13 = 80%;
jeweils nach 6 Tagen, während die bekannten Verbindungen (B), (C), (D) und (E) keine Wirkung zeigten.

### Beispiel D

### Myzus-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Keimlinge der Dicken Bohne (Vicia faba), die von der Grünen Pfirsichblattlaus (Myzus persicae) befallen sind, werden in die Wirkstoffzubereitung der gewünschten Konzentration getaucht und in eine Plastikdose gelegt.

Nach der gewünschten Zeit wird die Abtötung in Prozent bestimmt. Dabei bedeutet 100 %, daß alle Blattläuse abgetötet wurden; 0 % bedeutet, daß keine Blattläuse abgetötet wurden.

Bei diesem Test bewirkten bei einer beispielhaften Wirkstoffkonzentration von 0,1 % z.B. die Verbindungen folgender Herstellungsbeispiele folgende Abtötungen:
4 = 100 %; 15, 16, 17 = 90 % und 21 = 80 %;
jeweils nach 6 Tagen, während die bekannte Verbindung (A) keine Wirkung zeigte.

### Beispiel E

### Tetranychus-Test (OP-resistent/Tauchbehandlung)

| | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100%, daß alle Spinnmilben abgetötet wurden; 0% bedeutet, daß keine Spinnmilben abgetötet wurden.

Bei diesem Test bewirkten bei einer beispielhaften Wirkstoffkonzentration von 0,0001 % z.B. die Verbindungen der Herstellungsbeispiele 4, 5, 6, 9, 10, 12, 13, 15, 16, 17, 19, 20, 21, 22, 23, 24 und 25 eine Abtötung von 98 % jeweils nach 13 Tagen, während die bekannten Verbindungen (A) keine und (F) lediglich eine Abtötung von 45 % zeigten.

### Beispiel F

### Test mit Fliegenlarven / Entwicklungshemmende Wirkung

| | |
|---|---|
| Testtiere | Alle larvalen Stadien von Lucilia cuprina (OP-resistent) [Puppen und Adulte (ohne Kontakt zum Wirkstoff)] |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

30 - 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 Stunden) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 Stunden (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Flohentwicklung, bzw. ein Entwicklungsstillstand vor dem Adulten-Stadium. Dabei bedeutet 100 % larvizide Wirkung, daß nach 48 Stunden alle Larven abgestorben sind. 100 % entwicklungsinhibitorische Wirkung bedeutet, daß keine adulte Fliegen geschlüpft sind.

Bei diesem Test zeigten z.B. die Verbindungen gemäß den Herstellungsbeispielen 5 und 6 bei einer beispielhaften Wirkstoffkonzentration von 1000 ppm eine 100 %-ige Wirkung.

## Patentansprüche

1. Verbindungen der Formel (I) 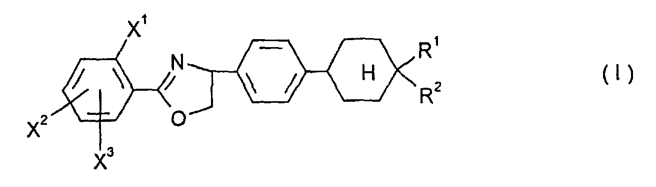 in welcher
X¹ für Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy, Amino, C₁-C₄-Alkylamino oder Di(C₁-C₄-alkyl)amino steht,
X² und X³ gleich oder verschieden sind und für Wasserstoff, Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy, Amino, C₁-C₄-Alkylamino oder Di(C₁-C₄-alkyl)amino stehen,
R¹ für Wasserstoff, -OR³ oder -SR³ steht und
R² für den Rest -OR⁴ steht,
oder
R¹ und R² gemeinsam für Sauerstoff, für =N-OR⁵ oder =N-NR⁶R⁷ stehen,
R³ und R⁴ gleich oder verschieden sind und für Wasserstoff, C₁-C₄-Alkyl oder gegebenenfalls, vorzugsweise im Phenylteil, einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl
oder gemeinsam mit den Heteroatomen, an die sie gebunden sind und dem diese verbindenden C-Atom, für einen gegebenenfalls einfach oder zweifach, gleich oder verschieden substituierten 5- oder 6-gliedrigen Ring stehen, wobei als Substituenten
gegebenenfalls durch Halogen, C₁-C₆-Alkoxy, C₂-C₆-Alkenyloxy, Benzyloxy oder Phenoxy (welche jeweils im Ring gegebenenfalls einfach bis dreifach, bevorzugt einfach oder zweifach, gleich oder verschieden durch Halogen, C₁-C₈-Alkyl, C₁-C₄-Alkoxy oder Phenoxy substituiert sind) substituiertes
C₁-C₁₈-Alkyl, C₂-C₆-Alkenyl, gegebenenfalls einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino, C₁-C₄-Alkoxy-carbonyl, Phenyl (welches gegebenenfalls einfach oder zweifach, gleich oder verschieden durch Halogen oder C₁-C₄-Alkyl substituiert ist) oder Benzyloxy (welches gegebenenfalls einfach oder zweifach, gleich oder verschieden im Ring durch Halogen oder C₁-C₄-Alkyl substituiert ist) substituiertes Phenyl oder -NR¹²R¹³ in Frage kommen,
R¹² für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil, einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxycarbonyl substituiertes Benzyl steht,
R¹³ für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxycarbonyl substituiertes Phenyl, Phenylcarbonyl oder Benzyl, für -CONR¹⁴R¹⁵ oder -SO₂NR¹⁴R¹⁵ steht,
R¹⁴ für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
R¹⁵ für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Phenyl, Phenylcarbonyl oder Benzyl steht,
R⁵ für Wasserstoff, C₁-C₆-Alkyl, C₂-C₆-Alkenyl, C₂-C₆-Alkinyl, C₁-C₄-Halogenalkyl oder gegebenenfalls, vorzugsweise im Phenylteil, einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
R⁶ für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Benzyl steht,
R⁷ für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Phenyl, Phenylcarbonyl oder Benzyl oder für -CONR⁸R⁹ oder -SO₂NR⁸R⁹ steht,
R⁸ für Wasserstoff, C₁-C₄-Alkyl, C₂-C₄-Alkenyl, C₂-C₄-Alkinyl, C₁-C₄-Halogenalkyl oder gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxycarbonyl substituiertes Benzyl steht und
R⁹ für C₁-C₄-Alkyl, C₁-C₄-Alkyl-carbonyl, C₁-C₄-Alkoxy-carbonyl, jeweils gegebenenfalls, vorzugsweise im Phenylteil einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Cyano, Nitro, Di(C₁-C₄-alkyl)amino oder C₁-C₄-Alkoxy-carbonyl substituiertes Phenyl, Phenylcarbonyl oder Benzyl steht.

2. Verbindungen der Formel (I) gemäß Anspruch 1, in welcher
X¹ für Fluor, Chlor oder Methyl steht,
X² und X³ gleich oder verschieden sind und für Wasserstoff, für Fluor, Chlor, Methyl, Ethyl, Methoxy, Methylthio, Trifluormethyl, Trifluormethoxy, Amino, Dimethylamino oder Diethylamino stehen,
R¹ für Wasserstoff, -OR³ oder -SR³ steht und
R² für den Rest -OR⁴ steht,
oder
R¹ und R² gemeinsam für Sauerstoff, für =N-OR⁵ oder =N-NR⁶R⁷ stehen,
R³ und R⁴ gleich oder verschieden sind und für Wasserstoff, Methyl, Ethyl, n- oder i-Propyl, n-, i-, s- oder t-Butyl; oder für gegebenenfalls, vorzugsweise im Phenylteil, einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl,
oder gemeinsam mit den Heteroatomen, an die sie gebunden sind, und dem diese verbindenden C-Atom für einen gegebenenfalls einfach bis dreifach, bevorzugt einfach oder zweifach, gleich oder verschieden substituierten 5- oder 6-gliedrigen Ring stehen, wobei als Substituenten
gegebenenfalls durch Fluor, Chlor, Methoxy, Ethoxy, n- oder i-Propoxy, n-Butyloxy, Allyloxy, Benzyloxy (welches jeweils im Ring gegebenenfalls einfach bis dreifach, bevorzugt einfach oder zweifach, gleich oder verschieden durch Fluor, Chlor, Brom, Methyl, Ethyl, Methoxy, Ethoxy oder Phenoxy substituiert ist) oder Phenoxy substituiertes C₁-C₁₈-Alkyl, C₂-C₄-Alkenyl, gegebenenfalls einfach bis dreifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl, Ethoxycarbonyl, Phenyl, Halogenphenyl, Benzyloxy oder Halogenbenzyloxy substituiertes Phenyl oder -NR¹²R¹³ in Frage kommen,
R¹² für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil, einfach bis dreifach, gleich oder verschieden durch Fluor, Chlor, Brom, Methyl, Ethyl, n- oder i-Propyl, n-, i-, s-oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl steht,
R¹³ für Methyl, Ethyl, n- oder i-Propyl, n-, i-, s- oder t-Butyl, Methylcarbonyl, Ethylcarbonyl, Methoxycarbonyl, Ethoxycarbonyl, für jeweils gegebenenfalls, vorzugsweise im Phenylteil, einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl, für -CONR¹⁴R¹⁵ oder -SO₂NR¹⁴R¹⁵ steht,
R¹⁴ für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil, einfach oder zweifach, gleich oder verschieden durch Fluor, Chlor, Brom, Methyl, Ethyl, n- oder i-Propyl, n-, i-, s-oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl steht,
R¹⁵ für Methyl, Ethyl, n- oder i-Propyl, n-, i-, s- oder t-Butyl, Methylcarbonyl, Ethylcarbonyl, Methoxycarbonyl, Ethoxycarbonyl, für jeweils gegebenenfalls, vorzugsweise im Phenylteil einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl steht,
R⁵ für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil einfach oder zweifach, gleich oder verschieden durch Fluor, Chlor, Brom, Methyl, Ethyl, n-oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl steht,
R⁶ für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil einfach oder zweifach, gleich oder verschieden durch Fluor, Chlor, Brom, Methyl, Ethyl, n-oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl steht,
R⁷ für Methyl, Ethyl, n- oder i-Propyl, n-, i-, s- oder t-Butyl, Methylcarbonyl, Ethylcarbonyl, Methoxycarbonyl, Ethoxycarbonyl, für jeweils gegebenenfalls, vorzugsweise im Phenylteil einfach oder zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl oder für -CONR⁸R⁹ oder -SO₂NR⁸R⁹ steht,
R⁸ für Wasserstoff, Methyl, Ethyl, n- oder i-Propyl, n-, i-, s- oder t-Butyl, Allyl, Propargyl, Trifluormethyl oder für gegebenenfalls, vorzugsweise im Phenylteil einfach bis dreifach, gleich oder verschieden durch Fluor, Chlor, Brom, Methyl, Ethyl, n-oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Benzyl steht und
R⁹ für Methyl, Ethyl, n- oder i-Propyl, n-, i-, s- oder t-Butyl, Methylcarbonyl, Ethylcarbonyl, Methoxycarbonyl, Ethoxycarbonyl, für jeweils gegebenenfalls, vorzugsweise im Phenylteil einfach bis zweifach, gleich oder verschieden durch Fluor, Chlor, Brom, Methyl, Ethyl, n- oder i-Propyl, n-, i-, s- oder t-Butyl; Methoxy, Ethoxy, Methylthio, Trifluormethyl, Trifluormethoxy, Trifluormethylthio, Cyano, Nitro, Dimethylamino, Diethylamino, Methoxycarbonyl oder Ethoxycarbonyl substituiertes Phenyl, Phenylcarbonyl oder Benzyl steht.

3. Verbindungen der Formel (I) gemäß Anspruch 1, in welcher
X¹ für Fluor, Chlor, C₁-C₂-Alkyl, C₁-C₂-Alkoxy oder C₁-C₂-Alkylthio steht,
X² für Wasserstoff, Fluor oder Chlor steht,
X³ für Wasserstoff, Fluor, Chlor oder C₁-C₂-Alkyl steht,
R¹ für den Rest -OR³ steht und
R² für den Rest -OR⁴ steht, wobei
R³ und R⁴ gemeinsam mit den O-Atomen, an die sie gebunden sind und dem diese verknüpfenden C-Atom für einen fünfgliedrigen Ring stehen, der gegebenenfalls substituiert sein kann durch C₁-C₁₈-Alkyl oder durch gegebenenfalls einfach bis dreifach, gleich oder verschieden durch Halogen, C₁-C₄-Alkyl, C₁-C₄-Alkoxy, C₁-C₄-Alkylthio, C₁-C₄-Halogenalkyl, C₁-C₄-Halogenalkoxy oder C₁-C₄-Halogenalkylthio substituiertes Phenyl.

4. Verbindungen der Formel (I) gemäß Anspruch 1, in welcher X¹, X², X³, R¹ und R² die in der folgenden Tabelle angegebenen Bedeutungen haben:

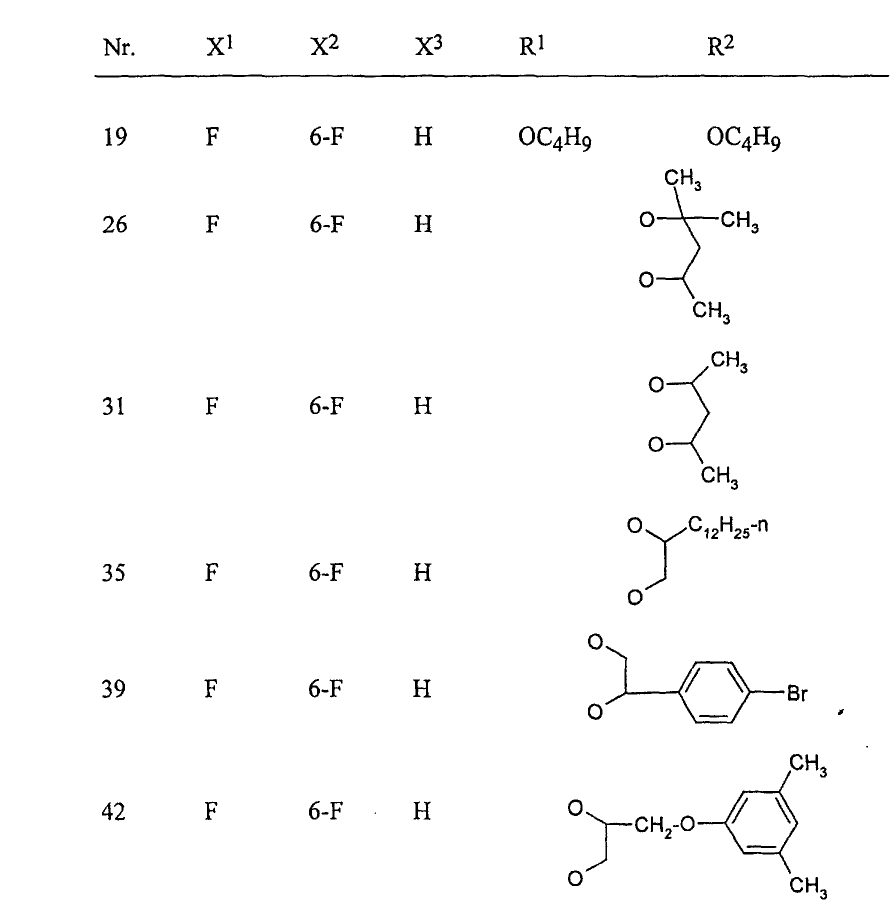

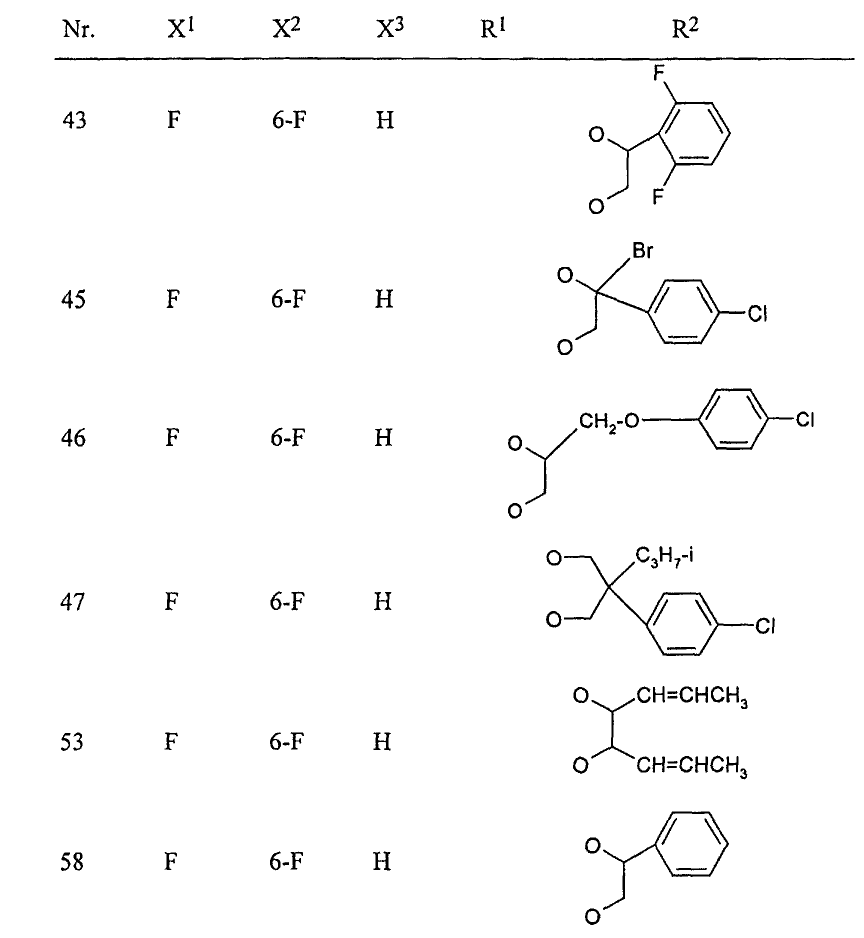

5. Verfahren zur Herstellung von Verbindungen der Formel (I) gemäß Anspruch 1, **dadurch gekennzeichnet, dass** man
a) Chlorethan-Derivate der Formel (II) 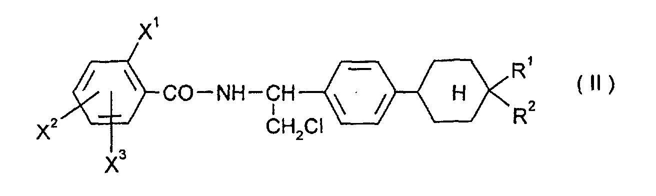 in welcher
X¹, X², X³, R¹ und R² die in Anspruch 1 angegebene Bedeutung haben,
in Gegenwart einer Base, gegebenenfalls in Gegenwart eines Katalysators und gegebenenfalls in Gegenwart eines Verdünnungsmittels cyclisiert;
oder
b) nach dem Verfahren (a) erhältliche 4-Cyclohexylphenyl-oxazoline der Formel (Ia) 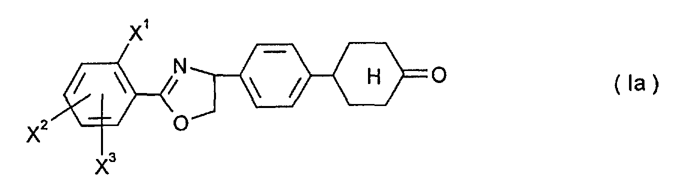 in welcher
X¹, X² und X³ die oben angegebene Bedeutung haben,
in üblicher und allgemein bekannter Art und Weise derivatisiert zu
(b1) Ketalen
durch Umsetzung mit Alkoholen der Formel (III), Thioalkoholen der Formel (IV) oder Verbindungen der Formel (V)
HOR¹⁰ (III)
HSR¹⁰ (IV)
HO-A-O(S)H (V)
in welchen
R¹⁰ für die oben genannten Bedeutungen von R³ und/oder R⁴ steht und
A für eine gegebenenfalls substituierte C₂- oder C₃-Kohlenwasserstoffkette steht,
gegebenenfalls in Gegenwart eines Katalysators und gegebenenfalls in Gegenwart eines Verdünnungsmittels;
oder
(b2) Oximen und Oximethern
durch Umsetzung mit Hydroxylaminen der Formel (VI)
H₂N-O-R⁵ (VI)
in welcher
R⁵ die in Anspruch 1 angegebene Bedeutung hat
oder mit deren Salzen, wie beispielsweise Halogenwasserstoff-Addukten gegebenenfalls in Gegenwart eines Verdünnungsmittels und gegebenenfalls in Gegenwart einer Base umsetzt; wobei es auch möglich ist, die entstehenden Derivate mit R⁵ = Wasserstoff nachträglich zu alkylieren;
oder
(b3) Hydrazonen
durch Umsetzung mit Hydrazinen der Formel (VII)
H₂N-NR⁶R⁷ (VII)
in welcher
R⁶ und R⁷ die in Anspruch 1 angegebene Bedeutung haben,
gegebenenfalls in Gegenwart eines Verdünnungsmittels;
oder
c) nach dem Verfahren (a) erhältliche 4-Cyclohexylphenyl-oxazoline der Formel (Ib) 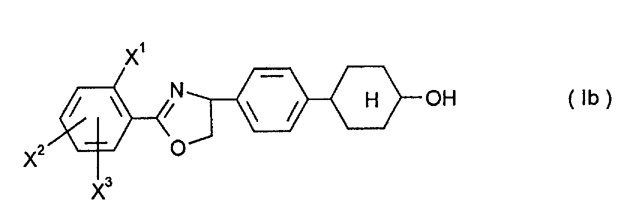 in welcher
X¹, X² und X³ die oben angegebene Bedeutung haben,
mit Halogeniden der Formel (VIII)
R¹¹-Hal (VIII)
in welcher
R¹¹ für Alkyl oder gegebenenfalls substituiertes Benzyl steht und
Hal für Halogen steht,
in Gegenwart einer Base und gegebenenfalls in Gegenwart eines Verdünnungsmittels umsetzt.

6. Verbindungen der Formel (II) 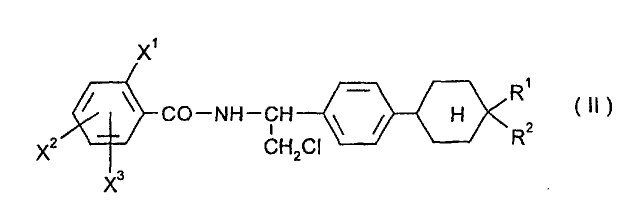 in welcher
X¹, X², X³, R¹ und R² die in Anspruch 1 angegebene Bedeutung haben.

7. Schädlingsbekämpfungsmittel, **gekennzeichnet durch** einen Gehalt an mindestens einer Verbindung der Formel (I) gemäß Anspruch 1.

8. Verwendung von Verbindungen der Formel (I) gemäß Anspruch 1 zur Bekämpfung von Schädlingen.

9. Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt.

10. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Mitteln vermischt.

11. Verwendung von Verbindungen der Formel (I) gemäß Anspruch 1 zur Herstellung von Schädlingsbekämpfungsmitteln.

## Claims

1. Compounds of the formula (I) 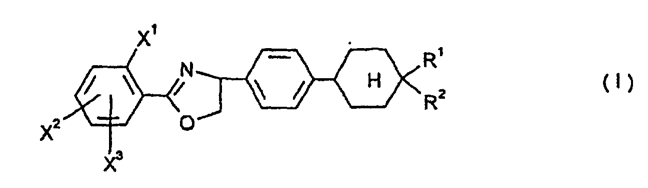 in which
X¹ represents fluorine, chlorine, bromine, C₁-C₄-alkyl, C₁-C₄-alkoxy, C₁-C₄-alkylthio, C₁-C₄-halogenoalkyl, C₁-C₄-halogenoalkoxy, amino, C₁-C₄-alkylamino or di(C₁-C₄-alkyl)amino,
X² and X³ are identical or different and each represents hydrogen, fluorine, chlorine, bromine, C₁-C₄-alkyl, C₁-C₄-alkoxy, C₁-C₄-alkylthio, C₁-C₄-halogenoalkyl, C₁-C₄-halogenoalkoxy, amino, C₁-C₄-alkylamino or di(C₁-C₄-alkyl)amino,
R¹ represents hydrogen, -OR³ or -SR³ and
R² represents the radical -OR⁴,
or
R¹ and R² together represent oxygen, represent =N-OR⁵ or =N-NR⁶R⁷,
R³ and R⁴ are identical or different and each represents hydrogen, C₁-C₄-alkyl or benzyl which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino and C₁-C₄-alkoxy-carbonyl
or they join with the hetero atoms to which they are attached and the carbon atom linking these to form a 5- or 6-membered ring which is optionally mono- or disubstituted by identical or different substitutents, suitable substituents being
C₁-C₁₈-alkyl which is optionally substituted by halogen, C₁-C₆-alkoxy, C₂-C₆-alkenyloxy, benzyloxy or phenoxy (each of which is optionally mono- to trisubstituted, preferably mono- or disubstituted, in the ring, by identical or different substituents from the group consisting of halogen, C₁-C₈-alkyl, C₁-C₄-alkoxy and phenoxy), C₂-C₆-alkenyl, phenyl which is optionally mono- to trisubstituted by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino, C₁-C₄-alkoxy-carbonyl, phenyl (which is optionally mono- or disubstituted by identical or different substituents from the group consisting of halogen and C₁-C₄-alkyl) and benzyloxy (which is optionally mono- or disubstituted in the ring by identical or different substituents from the group consisting of halogen and C₁-C₄-alkyl) and -NR¹²R¹³,
R¹² represents hydrogen, C₁-C₄-alkyl, C₂-C₄-alkenyl, C₂-C₄-alkynyl, C₁-C₄-halogenoalkyl or benzyl which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino and C₁-C₄-alkoxy-carbonyl,
R¹³ represents C₁-C₄-alkyl, C₁-C₄-alkyl-carbonyl, C₁-C₄-alkoxyl-carbonyl, represents phenyl, phenylcarbonyl or benzyl, each of which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino and C₁-C₄-alkoxy-carbonyl, represents -CONR¹⁴R¹⁵ or -SO₂NR¹⁴R¹⁵,
R¹⁴ represents hydrogen, C₁-C₄-alkyl, C₂-C₄-alkenyl, C₂-C₄-alkynyl, C₁-C₄-halogenoalkyl or benzyl which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino or C₁-C₄-alkoxy-carbonyl,
R¹⁵ represents C₁-C₄-alkyl, C₁-C₄-alkyl-carbonyl, C₁-C₄-alkoxy-carbonyl, represents phenyl, phenylcarbonyl or benzyl, each of which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino or C₁-C₄-alkoxy-carbonyl,
R⁵ represents hydrogen, C₁-C₆-alkyl, C₂-C₆-alkenyl, C₂-C₆-alkynyl, C₁-C₄-halogenoalkyl or benzyl which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino or C₁-C₄-alkoxy-carbonyl,
R⁶ represents hydrogen, C₁-C₄-alkyl, C₂-C₄-alkenyl, C₂-C₄-alkynyl, C₁-C₄-halogenoalkyl or benzyl which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino or C₁-C₄-alkoxy-carbonyl,
R⁷ represents C₁-C₄-alkyl, C₁-C₄-alkyl-carbonyl, C₁-C₄-alkoxy-carbonyl, represents phenyl, phenylcarbonyl or benzyl, each of which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino or C₁-C₄-alkoxy-carbonyl, or represents -CONR⁸R⁹ or -SO₂NR⁸R⁹,
R⁸ represents hydrogen, C₁-C₄-alkyl, C₂-C₄-alkenyl, C₂-C₄-alkynyl, C₁-C₄-halogenoalkyl or benzyl which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino or C₁-C₄-alkoxy-carbonyl and
R⁹ represents C₁-C₄-alkyl, C₁-C₄-alkyl-carbonyl, C₁-C₄-alkoxy-carbonyl, represents phenyl, phenylcarbonyl or benzyl, each of which is optionally mono- to trisubstituted, preferably in the phenyl moiety, by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, cyano, nitro, di(C₁-C₄-alkyl)amino or C₁-C₄-alkoxy-carbonyl.

2. Compounds of the formula (I) according to Claim 1 in which
X¹ represents fluorine, chlorine or methyl,
X² and X³ are identical or different and each represents hydrogen, represent fluorine, chlorine, methyl, ethyl, methoxy, methylthio, trifluoromethyl, trifluoromethoxy, amino, dimethylamino or diethylamino,
R¹ represents hydrogen, -OR³ or -SR³ and
R² represents the radical -OR⁴,
or
R¹ and R² together represent oxygen, represent =N-OR⁵ or =N-NR⁶R⁷,
R³ and R⁴ are identical or different and each represents hydrogen, methyl, ethyl, n- or i-propyl, n-, i-, s- or t-butyl; or represent benzyl which is optionally mono- or disubstituted, preferably in the phenyl moiety, by identical or different substituents from the group consisting of fluorine, chlorine, bromine, methyl, ethyl, n- or i-propyl, n-, i-, s- or t-butyl; methoxy, ethoxy, methylthio, trifluoromethyl, trifluoromethoxy, trifluoromethylthio, cyano, nitro, dimethylamino, diethylamino, methoxycarbonyl or ethoxycarbonyl,
or they join with the hetero atoms to which they are attached and the carbon atom linking these to form a 5- or 6-membered ring which is optionally mono- to trisubstituted, preferably mono- or disubstituted, by identical or different substituents, suitable substituents being
C₁-C₁₈-alkyl which is optionally substituted by fluorine, chlorine, methoxy, ethoxy, n- or i-propoxy, n-butyloxy, allyloxy, benzyloxy (which is in each case optionally mono- to trisubstituted, preferably mono- or disubstituted, in the ring, by identical or different substituents from the group consisting of fluorine, chlorine, bromine, methyl, ethyl, methoxy, ethoxy and phenoxy) and phenoxy, C₂-C₄-alkenyl, phenyl which is optionally mono- to trisubstituted by identical or different substituents from the group consisting of fluorine, chlorine, bromine, methyl, ethyl, n- or i-propyl, n-, i-, s- or t-butyl; methoxy, ethoxy, methylthio, trifluoromethyl, trifluoromethoxy, trifluoromethylthio, cyano, nitro, dimethylamino, diethylamino, methoxycarbonyl, ethoxycarbonyl, phenyl, halogenophenyl, benzyloxy and halogenobenzyloxy, and -NR¹²R¹³,
R¹² represents hydrogen, methyl, ethyl, n- or i-propyl, n-, i-, s-or t-butyl, allyl, propargyl, trifluoromethyl or represents benzyl which is optionally mono- to trisubstituted, preferably in the phenyl moiety, by identical or different substituents from the group consisting of fluorine, chlorine, bromine, methyl, ethyl, n- or i-propyl, n-, i-, s-or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl,
R¹³ represents methyl, ethyl, n- or i-propyl, n-, i-, s- or t-butyl, methylcarbonyl, ethylcarbonyl, methoxycarbonyl, ethoxycarbonyl, represents phenyl, phenylcarbonyl or benzyl, each of which is optionally mono- or disubstituted, preferably in the phenyl moiety, by identical or different substituents from the group consisting of fluorine, chlorine, bromine, methyl, ethyl, n- or i-propyl, n-, i-, s- or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl, represents -CONR¹⁴R¹⁵ or -SO₂NR¹⁴R¹⁵,
R¹⁴ represents hydrogen, methyl, ethyl, n- or i-propyl, n-, i-, s-or t-butyl, allyl, propargyl, trifluoromethyl or represents benzyl which is optionally mono- or disubstituted, preferably in the phenyl moiety, by identical or different substituents from the group consisting of fluorine, chlorine, bromine, methyl, ethyl, n- or i-propyl, n-, i-, s-or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl,
R¹⁵ represents methyl, ethyl, n- or i-propyl, n-, i-, s- or t-butyl, methylcarbonyl, ethylcarbonyl, methoxy-carbonyl, ethoxycarbonyl, represents phenyl, phenylcarbonyl or benzyl, each of which is optionally mono- or disubstituted, preferably in the phenyl moiety, by identical or different substituents from the group consisting of fluorine, chlorine, bromine, methyl, ethyl, n- or i-propyl, n-, i-, s- or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl,
R⁵ represents hydrogen, methyl, ethyl, n- or i-propyl, n-, i-, s- or t-butyl, allyl, propargyl, trifluoromethyl or represents benzyl which is optionally mono- or disubstituted, preferably in the phenyl moiety, by identical or different substituents from the group consisting of fluorine, chlorine, bromine, methyl, ethyl, n- or i-propyl, n-, i-, s- or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl,
R⁶ represents hydrogen, methyl, ethyl, n- or i-propyl, n-, i-, s- or t-butyl, allyl, propargyl, trifluoromethyl or represents benzyl which is optionally mono- or disubstituted, preferably in the phenyl moiety, by identical or different substituents from the group consisting of fluorine, chlorine, bromine, methyl, ethyl, n- or i-propyl, n-, i-, s- or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl,
R⁷ represents methyl, ethyl, n- or i-propyl, n-, i-, s- or t-butyl, methylcarbonyl, ethylcarbonyl, methoxycarbonyl, ethoxycarbonyl, represents phenyl, phenylcarbonyl or benzyl, each of which is optionally mono- or disubstituted, preferably in the phenyl moiety, by identical or different substituents from the group consisting of fluorine, chlorine, bromine, methyl, ethyl, n- or i-propyl, n-, i-, s-or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl, or represents -CONR⁸R⁹ or -SO₂NR⁸R⁹,
R⁸ represents hydrogen, methyl, ethyl, n- or i-propyl, n-, i-, s- or t-butyl, allyl, propargyl, trifluoromethyl or represents benzyl which is optionally mono- to trisubstituted, preferably in the phenyl moiety, by identical or different substituents from the group consisting of fluorine, chlorine, bromine, methyl, ethyl, n- or i-propyl, n-, i-, s- or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl,
R⁹ represents methyl, ethyl, n- or i-propyl, n-, i-, s- or t-butyl, methylcarbonyl, ethylcarbonyl, methoxycarbonyl, ethoxycarbonyl, represents phenyl, phenylcarbonyl or benzyl, each of which is optionally mono- to disubstituted, preferably in the phenyl moiety, by identical or different substituents from the group consisting of fluorine, chlorine, bromine, methyl, ethyl, n- or i-propyl, n-, i-, s-or t-butyl; methoxy, ethoxy, methylthio, trifluoromethyl, trifluoromethoxy, trifluoromethylthio, cyano, nitro, dimethylamino, diethylamino, methoxycarbonyl and ethoxycarbonyl.

3. Compounds of the formula (I) according to Claim 1 in which
X¹ represents fluorine, chlorine, C₁-C₂-alkyl, C₁-C₂-alkoxy or C₁-C₂-alkylthio,
X² represents hydrogen, fluorine or chlorine,
X³ represents hydrogen, fluorine, chlorine or C₁-C₂-alkyl,
R¹ represents the radical -OR³ and
R² represents the radical -OR⁴ where
R³ and R⁴ join with the oxygen atoms to which they are attached and the carbon atom linking these to form a five-membered ring which may be substituted by C₁-C₁₈-alkyl or by phenyl which is optionally mono- to trisubstituted by identical or different substituents from the group consisting of halogen, C₁-C₄-alkyl, C₁-C₄-alkoxy, C₁-C₄-alkylthio, C₁-C₄-halogenoalkyl, C₁-C₄-halogenoalkoxy and C₁-C₄-halogenoalkylthio.

4. Compounds of the formula (I) according to Claim 1 in which X¹, X², X³, R¹ and R² are each as defined in the table below:

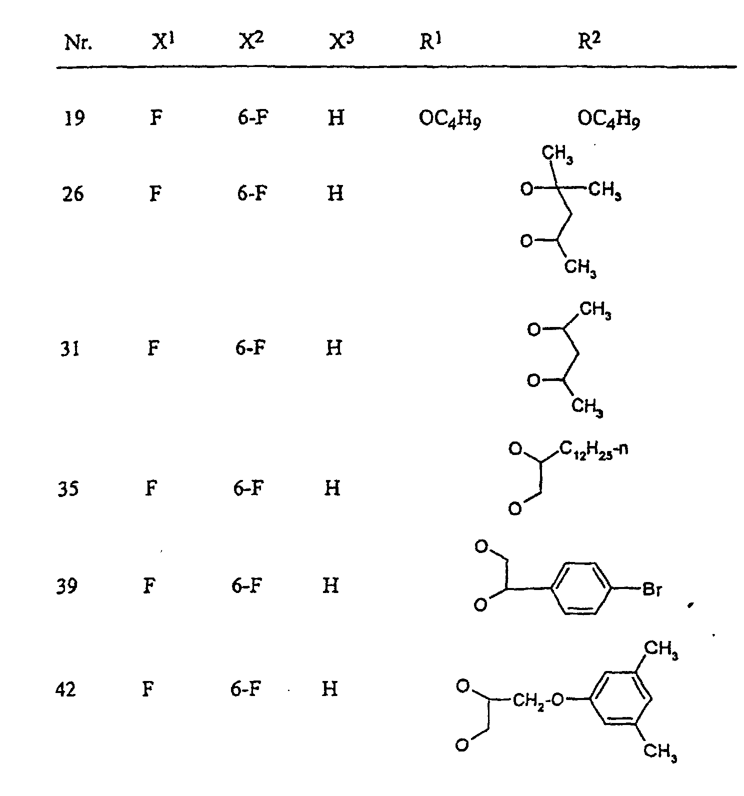

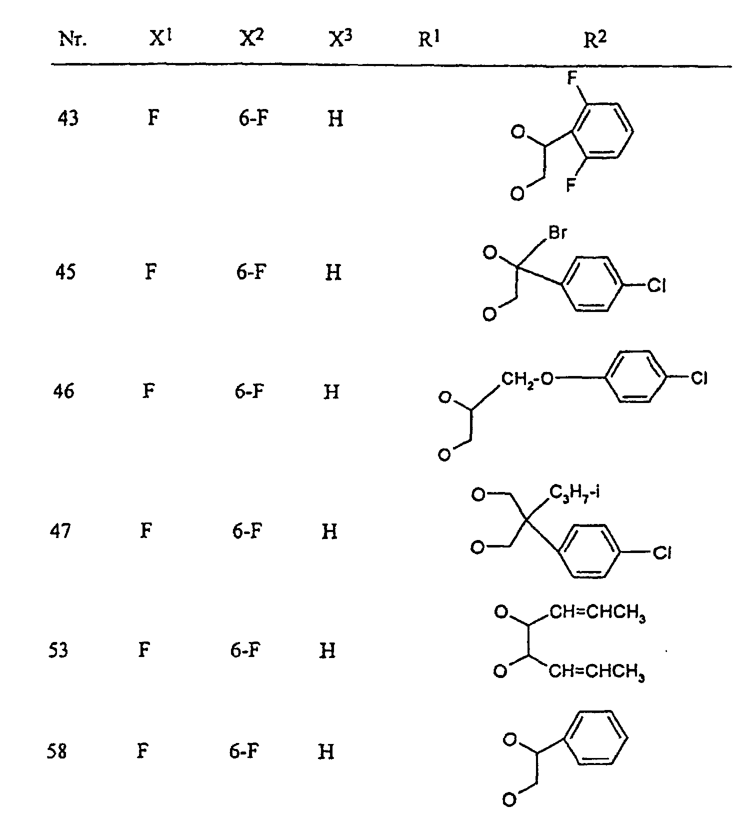

5. Process for preparing compounds of the formula (I) according to Claim 1, **characterized in that**
a) chloroethane derivatives of the formula (II) 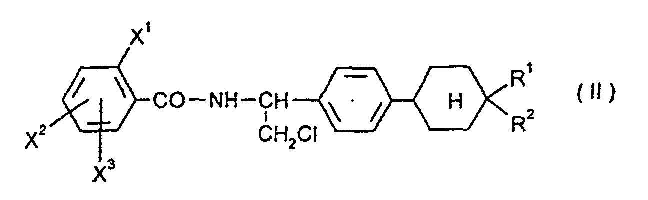 in which
X¹, X², X³, R¹ and R² are each as defined in Claim 1,
are cyclized in the presence of a base, if appropriate in the presence of a catalyst and if appropriate in the presence of a diluent;
or
b) 4-cyclohexylphenyl-oxazolines of the formula (Ia) 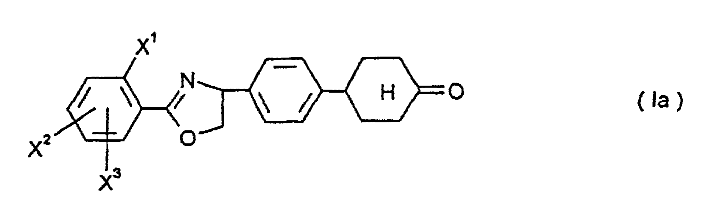 which are obtainable by the process (a) and in which
X¹, X² and X³ are each as defined above,
are derivatized in a conventional and generally known manner to give
(b1) ketals
by reaction with alcohols of the formula (III), thioalcohols of the formula (IV) or compounds of the formula (V)
HOR¹⁰ (III)
HSR¹⁰ (IV)
HO-A-O(S)H (V)
in which
R¹⁰ has the abovementioned meanings of R³ and/or R⁴ and
A represents an optionally substituted C₂- or C₃-hydrocarbon chain,
if appropriate in the presence of a catalyst and if appropriate in the presence of a diluent;
or
(b2) oximes and oxime ethers
by reaction with hydroxylamines of the formula (VI)
H₂N-O-R⁵ (VI)
in which
R⁵ is as defined in Claim 1
or with salts thereof such as, for example, hydrogen halide adducts, if appropriate in the presence of a diluent and if appropriate in the presence of a base; it is also possible to alkylate the resulting derivatives where R⁵ = hydrogen subsequently;
or
(b3) hydrazones
by reaction with hydrazines of the formula (VII)
H₂N-NR⁶R⁷ (VII)
in which
R⁶ and R⁷ are each as defined in Claim 1,
if appropriate in the presence of a diluent;
or
c) 4-cyclohexylphenyl-oxazolines of the formula (Ib) 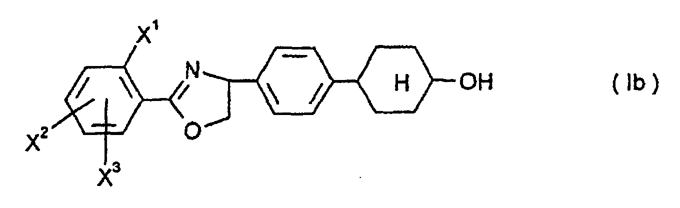 which are obtainable by process (a) and in which
X¹, X² and X³ are each as defined above,
are reacted with halides of the formula (VIII)
R¹¹-Hal (VIII)
in which
R¹¹ represents alkyl or optionally substituted benzyl and
Hal represents halogen,
in the presence of a base and if appropriate in the presence of a diluent.

6. Compounds of the formula (II) 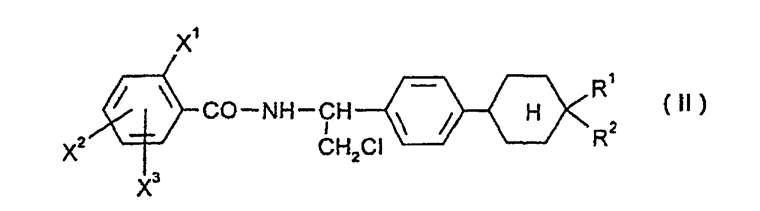 in which
X¹, X², X³, R¹ and R² are each as defined in Claim 1.

7. Pesticides, **characterized in that** they contain at least one compound of the formula (I) according to Claim 1.

8. Use of compounds of the formula (I) according to Claim 1 for controlling pests.

9. Method for controlling pests, **characterized in that** compounds of the formula (I) according to Claim 1 are allowed to act on pests and/or their habitat.

10. Process for preparing pesticides, **characterized in that** compounds of the formula (I) according to Claim I are mixed with extenders and/or surfactants.

11. Use of compounds of the formula (I) according to Claim 1 for preparing pesticides.

## Revendications

1. Composés de formule (I) 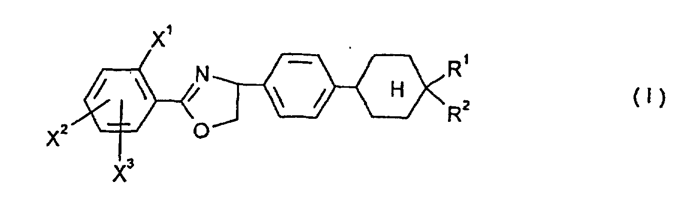 dans laquelle
X¹ représente le fluor, le chlore, le brome, un groupe alkyle en C₁ à C₄, alkoxy en C₁ à C₄, alkylthio en C₁ à C₄, halogénalkyle en C₁ à C₄, halogénalkoxy en C₁ à C₄, amino, alkylamino en C₁ à C₄ ou di(alkyle en C₁ à C₄)amino,
X² et X³ sont identiques ou différents et représentent l'hydrogène, le fluor, le chlore, le brome, un reste alkyle en C₁ à C₄, alkoxy en C₁ à C₄, alkylthio en C₁ à C₄, halogénalkyle en C₁ à C₄, halogénalkoxy en C₁ à C₄, amino, alkylamino en C₁ à C₄ ou di(alkyle en C₁ à C₄)-amino,
R¹ représente l'hydrogène, un reste -OR³ ou -SR³ et
R² représente le reste -OR⁴,
ou bien
R¹ et R² représentent ensemble l'oxygène, un reste =N-OR⁵ ou =N-NR⁶R⁷,
R³ et R⁴ sont identiques ou différents et représentent l'hydrogène, un reste alkyle en C₁ à C₄ ou un reste benzyle portant éventuellement, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄)carbonyle,
ou forment conjointement avec les hétéroatomes auxquels ils sont liés et avec l'atome de carbone liant ces hétéroatomes, un noyau pentagonal ou hexagonal portant éventuellement un ou deux substituants identiques ou différents, les substituants considérés étant un reste alkyle en C₁ à C₁₈ portant éventuellement un substituant halogéno, alkoxy en C₁ à C₆, alcényloxy en C₂ à C₆, benzyloxy ou phénoxy (dont chacun est éventuellement substitué dans le noyau une à trois fois, de préférence une ou deux fois identiques ou différentes, par un radical halogéno, alkyle en C₁ à C₈, alkoxy en C₁ à C₄ ou phénoxy), un reste alcényle en C₂ à C₆, un reste phényle portant éventuellement un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino, (alkoxy en C₁ à C₄)carbonyle, phényle (qui porte éventuellement un ou deux substituants halogéno ou alkyle en C₁ à C₄ identiques ou différents) ou benzyloxy (qui porte éventuellement dans le noyau un ou deux substituants halogéno ou alkyle en C₁ à C₄ identiques ou différents) ou un reste -NR¹²R¹³,
R¹² représente l'hydrogène, un reste alkyle en C₁ à C₄, alcényle en C₂ à C₄, alcynyle en C₂ à C₄, halogénalkyle en C₁ à C₄ ou benzyle portant éventuellement, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₉, cyano, nitro, di(alkyle en C₁ à C₄)amino ou (alkoxy en C₁ à C₄)carbonyle,
R¹³ est un reste alkyle en C₁ à C₄, (alkyle en C₁ à C₄)carbonyle, (alkoxy en C₁ à C₄)carbonyle, un reste phényle, phénylcarbonyle ou benzyle dont chacun est éventuellement substitué, de préférence dans la partie phényle, une à trois fois identiques ou différentes par un radical halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄)carbonyle, un reste -CONR¹⁴R¹⁵ ou un reste -SO₂NR¹⁴R¹⁵,
R¹⁴ représente l'hydrogène, un reste alkyle en C₁ à C₄, alcényle en C₂ à C₄, alcynyle en C₂ à C₄, halogénalkyle en C₁ à C₄ ou un reste benzyle portant éventuellement, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄)-carbonyle,
R¹⁵ est un reste alkyle en C₁ à C₄ (alkyle en C₁ à C₄)carbonyle, (alkoxy en C₁ à C₄)carbonyle, un reste phényle, phénylcarbonyle ou benzyle, dont chacun porte éventuellement, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di-(alkyle en C₁ à C₄)amino ou (alkoxy en C₁ à C₄)-carbonyle,
R⁵ représente l'hydrogène, un reste alkyle en C₁ à C₆, alcényle en C₂ à C₆, alcynyle en C₂ à C₆, halogénalkyle en C₁ à C₄ ou un reste benzyle portant éventuellement, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄)carbonyle,
R⁶ représente l'hydrogène, un reste alkyle en C₁ à C₄, alcényle en C₂ à C₄, alcynyle en C₂ à C₄, halogénalkyle en C₁ à C₄ ou un reste benzyle portant éventuellement, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄)carbonyle,
R⁷ est un reste alkyle en C₁ à C₄, (alkyle en C₁ à C4)-carbonyle, (alkoxy en C₁ à C₄)carbonyle, un reste phényle, phénylcarbonyle ou benzyle dont chacun porte, le cas échéant, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄)carbonyle ou un reste -CONR⁸R⁹ ou -SO₂NR⁸R⁹,
R⁸ représente l'hydrogène, un reste alkyle en C₁ à C₄, alcényle en C₂ à C₄, alcynyle en C₂ à C₄, halogénalkyle en C₁ à C₄ ou un reste benzyle portant, le cas échéant, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄)carbonyle, et
R⁹ est un reste alkyle en C₁ à C₄, (alkyle en C₁ à C₄)carbonyle, (alkoxy en C₁ à C₄)carbonyle, un reste phényle, phénylcarbonyle ou benzyle portant chacun, le cas échéant, de préférence dans la partie phényle, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, cyano, nitro, di(alkyle en C₁ à C₄)amino ou (alkoxy en C₁ à C₄) carbonyle.

2. Composés suivant la revendication 1, de formule (I) dans laquelle
X¹ représente le fluor, le chlore ou le reste méthyle,
X² et X³ sont identiques ou différents et représentent l'hydrogène, le fluor, le chlore, un reste méthyle, éthyle, méthoxy, méthylthio, trifluorométhyle, trifluorométhoxy, amino, diméthylamino ou diéthylamino,
R¹ est l'hydrogène, un reste -OR³ ou -SR³ et
R² est le reste -OR⁴,
ou bien
R¹ et R² représentent conjointement l'oxygène, un reste =N-OR⁵ ou =N-NR⁶R⁷,
R³ et R⁴ sont identiques ou différents et représentent l'hydrogène, un reste méthyle, éthyle, n-propyle, isopropyle, n-butyle, isobutyle, sec.-butyle, tertio-butyle ; ou un reste benzyle portant éventuellement, de préférence dans la partie phényle, un ou deux substituants, identiques ou différents, fluoro, chloro, bromo, méthyle, éthyle, n-propyle, isopropyle, n-butyle, iso-butyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle, ou forment conjointement avec les hétéroatomes auxquels ils sont liés et avec l'atome de carbone liant ces hétéroatomes, un noyau pentagonal ou hexagonal portant éventuellement un à trois substituants, de préférence un ou deux substituants, identiques ou différents, les substituants que l'on considère étant
un reste alkyle en C₁ à C₁₈ portant éventuellement un substituant fluoro, chloro, méthoxy, éthoxy, n-propoxy, isopropoxy, n-butyloxy, allyloxy, benzyloxy (qui porte dans le noyau, le cas échéant, un à trois, de préférence un ou deux, substituants, identiques ou différents, fluoro, chloro, bromo, méthyle, éthyle, méthoxy, éthoxy ou phénoxy) ou phénoxy, un reste alcényle en C₂ à C₄, un reste phényle portant éventuellement un à trois substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle, éthoxycarbonyle, phényle, halogénophényle, benzyloxy ou halogénobenzyloxy ou un reste -NR¹²R¹³,
R¹² représente l'hydrogène, un reste méthyle, éthyle, n-propyle, isopropyle, n-butyle, iso-butyle, sec.-butyle, tertio-butyle, allyle, propargyle, trifluorométhyle ou un reste benzyle portant éventuellement, de préférence dans la partie phényle, un à trois substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluoro-méthoxy, trifluorométhylthio, cyano, nitro, diméthylamino, diéthylamino, méthoxycarbonyle ou éthoxycarbonyle,
R¹³ est un reste méthyle, éthyle, n-propyle, isopropyle, n-butyle, isobutyle, sec.-butyle, tertio-butyle, méthylcarbonyle, éthylcarbonyle, méthoxycarbonyle, éthoxycarbonyle, un reste phényle, phénylcarbonyle ou benzyle, chacun portant éventuellement, de préférence dans la partie phényle, un ou deux substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle, un reste -CONR¹⁴R¹⁵ ou un reste -SO₂NR¹⁴R¹⁵,
R¹⁴ représente l'hydrogène, un reste méthyle, éthyle, n-propyle, isopropyle, n-butyle, isobutyle, sec.-butyle, tertio-butyle, allyle, propargyle, trifluorométhyle ou un reste benzyle portant éventuellement, de préférence dans la partie phényle, un à trois substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle,
R¹⁵ est un reste méthyle, éthyle, n-propyle, isopropyle, n-butyle, isobutyle, sec.-butyle, tertio-butyle, méthylcarbonyle, éthylcarbonyle, méthoxycarbonyle, éthoxycarbonyle, un reste phényle, phénylcarbonyle ou benzyle, portant chacun, le cas échéant, de préférence dans la partie phényle, un ou deux substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle,
R⁵ représente l'hydrogène, un reste méthyle, éthyle, n-propyle, isopropyle, n-butyle, isobutyle, sec.-butyle, tertio-butyle, allyle, propargyle, trifluorométhyle ou un reste benzyle portant éventuellement, de préférence dans la partie phényle, un ou deux substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle,
R⁶ représente l'hydrogène, un reste méthyle, éthyle, n-propyle, isopropyle, n-butyle, isobutyle, sec.-butyle, tertio-butyle, allyle, propargyle, trifluorométhyle ou un reste benzyle portant éventuellement, de préférence dans la partie phényle, un ou deux substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle,
R⁷ est un reste méthyle, éthyle, n-propyle, isopropyle, n-butyle, isobutyle, sec.-butyle, tertio-butyle, méthylcarbonyle, éthylcarbonyle, méthoxycarbonyle, éthoxycarbonyle, un reste phényle, phénylcarbonyle ou benzyle, chacun portant, le cas échéant, de préférence dans la partie phényle, un ou deux substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle, ou un reste -CONR⁸R⁹ ou -SO₂NR⁸R⁹,
R⁸ représente l'hydrogène, un reste méthyle, éthyle, n-propyle, isopropyle, n-butyle, isobutyle, sec.-butyle, tertio-butyle, allyle, propargyle, trifluorométhyle ou un reste benzyle portant éventuellement, de préférence dans la partie phényle, un à trois substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle, et
R⁹ est un méthyle, éthyle, n-propyle, isopropyle, n-butyle, isobutyle, sec.-butyle, tertio-butyle, méthylcarbonyle, éthylcarbonyle, méthoxycarbonyle, éthoxycarbonyle, un reste phényle, phénylcarbonyle ou benzyle, chacun portant, le cas échéant, de préférence dans la partie phényle, un ou deux substituants, identiques ou différents, fluoro, chloro, bromo, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, cyano, nitro, diméthylamino, diéthylamino, méthoxycarbonyle ou éthoxycarbonyle.

3. Composés suivant la revendication 1, de formule (I) dans laquelle
X¹ représente le fluor, le chlore, un reste alkyle en C₁ ou C₂, alkoxy en C₁ ou C₂ ou alkylthio en C₁ ou C₂,
X² représente l'hydrogène, le fluor ou le chlore,
X³ représente l'hydrogène, le fluor, le chlore ou un reste alkyle en C₁ ou C₂,
R¹ représente le reste -OR³ et
R² représente le reste -OR⁴,
R³ et R⁴ formant conjointement avec les atomes d'azote auxquels ils sont liés et avec l'atome de carbone liant ces atomes, un noyau pentagonal qui peut éventuellement porter un substituant alkyle en C₁ à C₁₈ ou, le cas échéant, un à trois substituants, identiques ou différents, halogéno, alkyle en C₁ à C₄, alkoxy en C₁ à C₄, alkylthio en C₁ à C₄, halogénalkyle en C₁ à C₄, halogénalkoxy en C₁ à C₄ ou halogénalkylthio en C₁ à C₄.

4. Composés suivant la revendication 1, de formule (I) dans laquelle X¹, X², X³, R¹ et R² ont les définitions indiquées dans le tableau suivant :

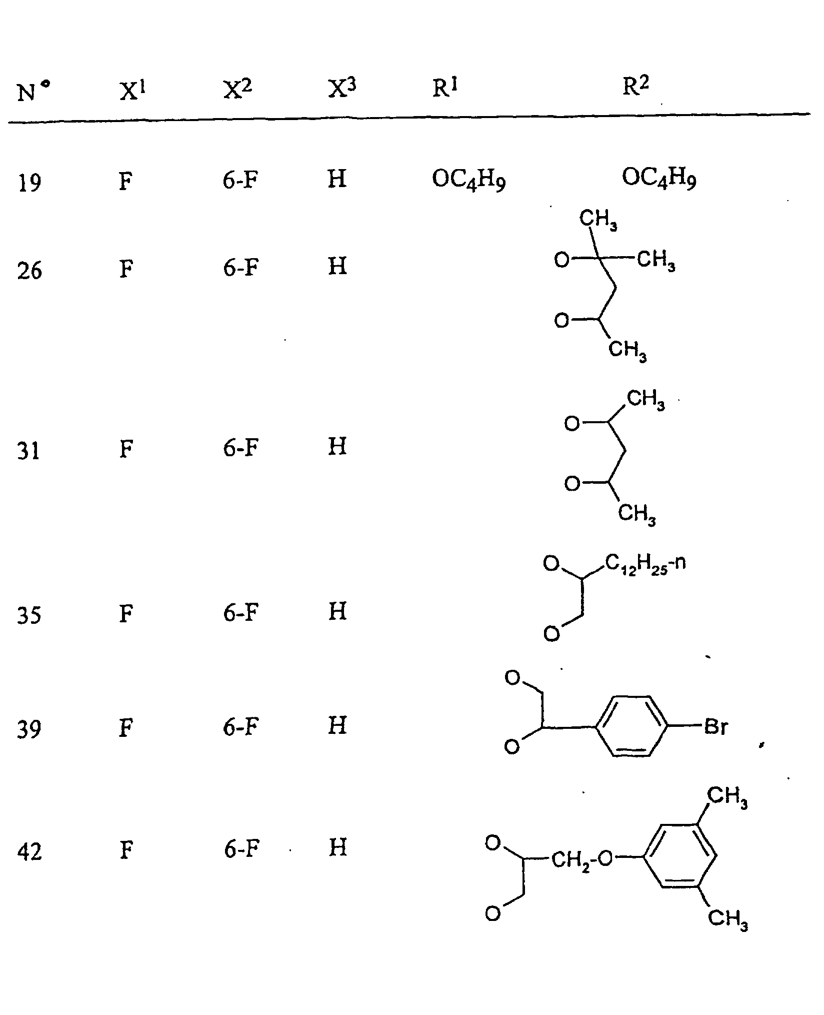

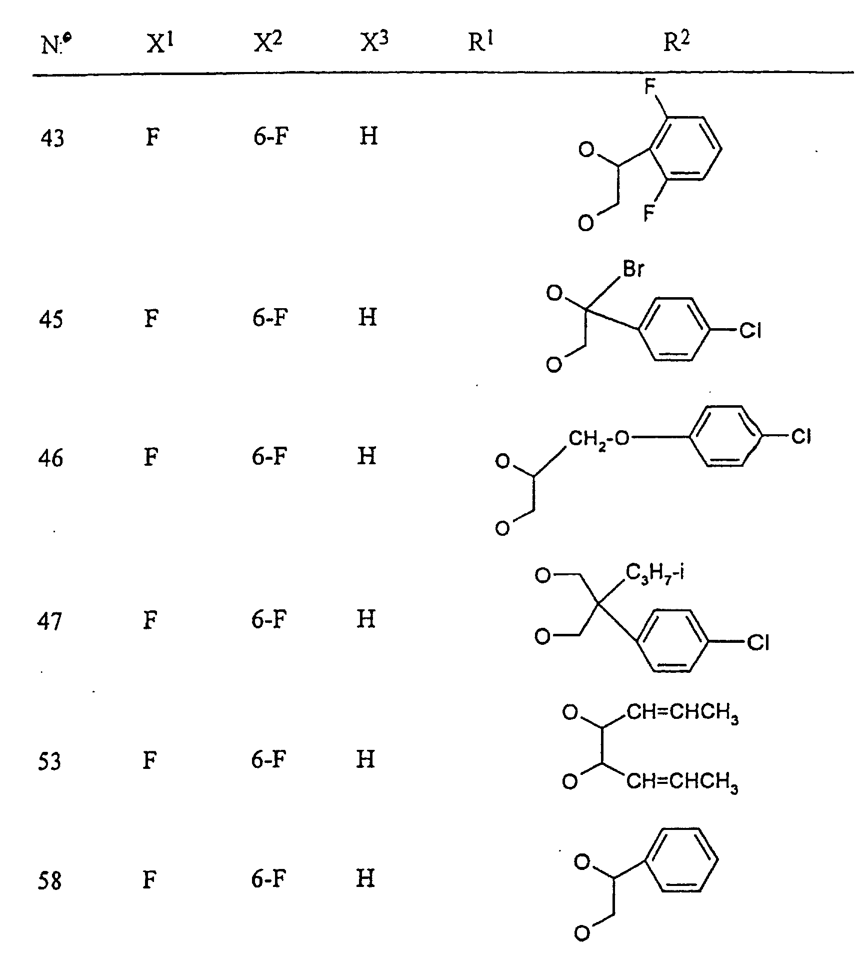

5. Procédé de production de composés de formule (I) suivant la revendication 1, **caractérisé en ce que** :
a) on cyclise des dérivés de chloréthane de formule (II) 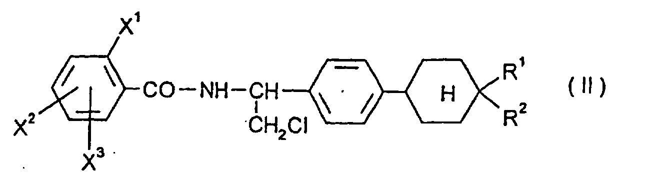 dans laquelle
X¹, X², X³, R¹ et R² ont la définition indiquée dans la revendication 1,
en présence d'une base, éventuellement en présence d'un catalyseur et, le cas échéant, en présence d'un diluant ;
ou bien
b) on transforme en dérivé d'une manière classique et généralement connue des 4-cyclohexylphényl-oxazolines obtenues selon le procédé (a) de formule (Ia) 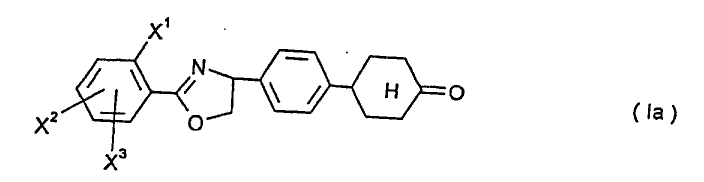 dans laquelle
X¹, X² et X³ ont la définition indiquée ci-dessus, pour former
(b1) des cétals
par réaction avec des alcools de formule (III), des thio-alcools de formule (IV) ou des composés de formule (V)
HOR¹⁰ (III)
HSR¹⁰ (IV)
HO-A-O(S)H (V)
formules dans lesquelles
R¹⁰ a les définitions indiquées ci-dessus pour R³ et/ou R⁴ et
A est une chaîne hydrocarbonée en C₂ ou C₃ éventuellement substituée,
le cas échéant en présence d'un catalyseur et en présence éventuelle d'un diluant ;
ou bien
(b2) des oximes et des éthers d'oximes
par réaction avec des hydroxylamines de formule (VI)
H₂N-OR⁵ (VI)
dans laquelle
R⁵ a la définition indiquée dans la revendication 1,
ou avec leurs sels tels que, par exemple, les produits d'addition d'hydracides halogénés, éventuellement en présence d'un diluant et, le cas échéant en présence d'une base, et il est alors également possible d'alkyler ultérieurement les dérivés produits dans lesquels R⁵ est l'hydrogène ;
ou
(b3) des hydrazones
par réaction avec des hydrazines de formule (VII)
H₂N-NR⁶R⁷ (VII)
dans laquelle
R⁶ et R⁷ ont la définition indiquée dans la revendication 1,
le cas échéant en présence d'un diluant ;
ou bien
c) on fait réagir des 4-cyclohexylphényl-oxazolines obtenues selon le procédé (a), de formule (Ib) 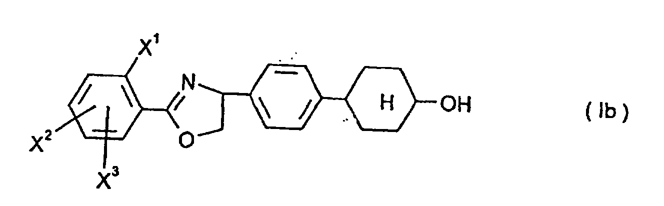
dans laquelle
X¹, X² et X³ ont la définition indiquée ci-dessus,
avec des halogénures de formule (VIII)
dans laquelle
R¹¹ est un reste alkyle ou un reste benzyle éventuellement substitué et
Hal représente un halogène,
en présence d'une base et, le cas échéant en présence d'un diluant.

6. Composés de formule (II) 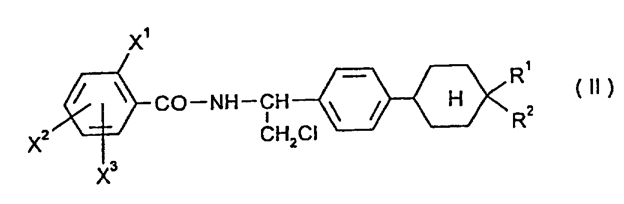 dans laquelle
X¹, X², X³, R¹ et R² ont la définition indiquée dans la revendication 1.

7. Compositions pesticides, **caractérisées par** une teneur en au moins un composé de formule (I) suivant la revendication 1.

8. Utilisation de composés de formule (I) suivant la revendication 1 pour combattre des parasites.

9. Procédé pour combattre des parasites, **caractérisé en ce qu'**on fait agir des composés de formule (I) suivant la revendication 1 sur les parasites et/ou sur leur milieu.

10. Procédé de préparation de compositions pesticides, **caractérisé en ce qu'**on mélange des composés de formule (I) suivant la revendication 1 avec des diluants et/ou des agents tensio-actifs.

11. Utilisation de composés de formule (I) suivant la revendication 1 pour la préparation de compositions pesticides.
